(19) **Europäisches Patentamt European Patent Office Office européen des brevets**

(11) **EP 2 921 136 B1**

(12) **EUROPEAN PATENT SPECIFICATION**

(45) Date of publication and mention of the grant of the patent:
**23.10.2019 Bulletin 2019/43**

(51) Int Cl.:
*A61L 31/14* (2006.01)    *A61L 31/12* (2006.01)
*A61L 31/16* (2006.01)    *B29C 48/08* (2019.01)
*D01D 5/00* (2006.01)    *B29K 27/00* (2006.01)
*B29K 75/00* (2006.01)    *B29L 31/00* (2006.01)
*B29K 23/00* (2006.01)    *A61L 31/04* (2006.01)
*A61F 2/00* (2006.01)

(21) Application number: **13854718.7**

(22) Date of filing: **14.11.2013**

(86) International application number:
**PCT/CN2013/001387**

(87) International publication number:
**WO 2014/075388 (22.05.2014 Gazette 2014/21)**

(54) **FIBER MEMBRANES FOR REPAIRING TISSUE AND PRODUCTS AND PREPARATION METHOD THEREOF**

FASERMEMBRANEN ZUR REPARATUR VON GEWEBE SOWIE PRODUKTE UND HERSTELLUNGSVERFAHREN DAFÜR

MEMBRANES À FIBRE POUR LA RÉPARATION DE TISSU ET DE PRODUITS, ET LEUR PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.11.2012 CN 201210457521**
**14.11.2012 CN 201210457158**
**23.08.2013 CN 201310372804**

(43) Date of publication of application:
**23.09.2015 Bulletin 2015/39**

(73) Proprietor: **Medprin Regenerative Medical Technologies Co., Ltd.**
**Guangzhou, Guangdong 510663 (CN)**

(72) Inventors:
• **GUO, Zeyue**
**Guangzhou**
**Guangdong 510663 (CN)**
• **LAI, Kuilin**
**Guangzhou**
**Guangdong 510663 (CN)**
• **WANG, Guoshuai**
**Guangzhou**
**Guangdong 510663 (CN)**
• **ZHAN, Zefeng**
**Guangzhou**
**Guangdong 510663 (CN)**
• **XU, Tao**
**Guangzhou**
**Guangdong 510663 (CN)**
• **YUAN, Yuyu**
**Guangzhou**
**Guangdong 510663 (CN)**

(74) Representative: **Zeitler Volpert Kandlbinder Patent- und Rechtsanwälte Partnerschaft mbB**
**Herrnstrasse 44**
**80539 München (DE)**

(56) References cited:
**WO-A1-2011/003422**    **WO-A2-2008/008266**
**CN-A- 101 507 661**    **CN-A- 101 692 986**

• LI, XINSONG ET AL.,: 'NANO-POROUS ULTRA-HIGH SPECIFIC SURFACE ULTRAFINE FIBERS' CHINESE SCIENCE BULLETIN vol. 49, no. 21, November 2004, pages 2160 - 2163, XP008178960

- **YAO, XIANG ET AL.,: 'PREPARING BIODEGRADABLE POLYURETHANE POROUS SCAFFOLD FOR TISSUE ENGINEERING APPLICATION' PROGRESS IN CHEMISTRY vol. 21, no. 7/8, 24 August 2009, pages 1546 - 1552, XP055248561**

**Description**

**Technical Field**

The present invention relates to a fibrous membrane used for tissue repair and preparation methods and applications thereof as well as products used for tissue repair.

**Background Art**

Tissue and organ repair is a medical problem in the whole world. In modern surgeries, the repair for tissues and organs is often needed, such as hernia repair, treatment of female pelvic floor dysfunction, dura/spinal meninges coloboma repair, nerve conduit coloboma repair, blood vessel repair, ligament repair, bone coloboma repair, etc. Due to the particularity of human tissue structures and functions, the ideal fibrous membrane used for tissue repair should possess the following properties: (1) having certain strength which is resistant to mechanical stress, and able to withstand the intensity of pressure within the abdominal cavity before healthy tissue is completely formed; (2) facilitating cell adhesion, crawling and growth to achieve tissue regeneration; (3) preventing bacteria from hiding and breeding to avoid infection caused by using artificial biological synthetic materials; (4) after being implanted into a human body, keeping good dimensional stability, without shrinkage or deformation; (5) being stable in structure, convenient to be cut into required shapes without decoherence, and convenient for suture with tissue; (6) being soft in handfeel and convenient to form, thereby improving the maneuverability of the surgeries, reducing the discomfort of patients, and enhancing the effect of the surgeries; and (7) having good biocompatibility, and being capable of guiding tissue growth to achieve ideal repair.

Patent WO 2011/003422 A1 discloses a biodegradable surgical implant comprising a) a synthetic biodegradable homogenous sheet of scaffold, b) one or more biodegradable reinforcing members. The sheet of scaffold is obtained by electrospinning and exhibits a tensile strength in the range of about 5-40 psi, has an open pore structure with a size in the range of 30 - 200 $\mu$m and is used to treat pelvic organ prolapse.

The existing fibrous membrane used for tissue repair generally has the following problems:

1) A woven mesh is most widely used in the fibrous membrane used for tissue repair, but this kind of product has rough surface, relatively hard texture and poor biocompatibility, and is easy to produce foreign body sensation and pain, causing common complications such as erosion and infection; moreover, immunological rejection caused by this material is stronger, so that more sequelae of the surgeries exist; and when being in direct contact with viscera and organs, it is easy to cause damage, and can cause relatively serious adhesion, triggering serious foreign body and immune responses, therefore a second surgery is needed for taking it out, which brings pain to the patient, and even threatens his life.

2) An electrospinning membrane under the current study often has the defect that cells are difficult to grow into it or growth is slow.

Thus it can be seen, the performance of the existing fibrous membrane used for tissue repair which is formed by using a weaving or electrospinning technology is not ideal.

**Disclosure of the Invention**

Problems to be solved by the invention

The present invention is made in view of the above problems of the prior art. The purpose of the invention is to provide a fibre membrane used for tissue repair which has good mechanical strength, can provide enough mechanical support before complete repair, is conducive to cell adhesion and proliferation, guides cell differentiation, facilitates tight suture between tissues, reduces the occurence of shrinkage of the products used for tissue repair, caused infection, adhesion with viscera and organs, etc., to improve the patient's comfort, and achieve repair quickly to reduce the pain of the patient.

On the other hand, the purpose of the invention is also to provide an implantable membrane used for tissue repair which has good softness and excellent biocompatibility, is conducive to tissue ingrowth in order to form firm repair, and can further have anti-infective and hemostatic properties.

Further, the purpose of the invention is to provide an anti-adhesion tissue repair membrane which can also effectively prevent repaired tissue from adhering with surrounding tissue.

Means for solving the problems

The present invention provides a fibrous membrane used for tissue repair as disclosed in claim 1, which is characterized in that the fibrous membrane is formed by interweaving fibrous filaments with diameters of 10nm-100$\mu$m, and has a porous structure, with the fluffiness of 200-2,000cm$^3$/g, so as to solve the above problems.
The average pore size of the fibrous membrane is 50-500$\mu$m, the thickness is 0.2-2mm, the tensile strength is 10-300N/cm, and the softness is 50-500mN.
The present invention also provides an implantable membrane used for tissue repair for treating female pelvic floor dysfunction which comprises the fibrous membrane used for tissue repair, and an anti-adhesion tissue repair membrane which comprises the fibrous membrane used for tissue repair.
In addition, present invention also provides preparation methods and applications of the fibrous membrane used for tissue repair, the implantable membrane used for tissue repair and the anti-adhesion tissue repair membrane.

Effects of the Invention

Compared with the prior art, the invention has the following beneficial effects:

(1) The fibrous membrane used for tissue repair provided by the invention has large specific surface area, thus being conducive to cell adhesion and proliferation, and a topological structure on the surface of fiber also helps to guide cell differentiation;
(2) The fibrous membrane used for tissue repair provided by the invention has a porous fluffy structure which is more conducive to rapid ingrowth of fibroblasts compared with the structure of a common electrospinning membrane, so as to have strengthening and fixing effects, and achieve a rapid and ideal repair effect;
(3) The fibrous membrane used for tissue repair provided by the invention is light and soft in texture, thereby being more conducive to tight suture with tissues, and improving the patient's comfort;
(4) The fibrous membrane used for tissue repair provided by the invention can reduce the occurence of shrinkage of the products used for repair, caused infection, adhesion with tissues such as viscera and organs, etc.;
(5) As the fibrous membrane used for tissue repair provided by the invention does not contain components of living cells, sources of materials are sufficient, costs are relatively low, insufficient sources of autologous and allogenic materials as well as materials of animal origin are avoided, and storage and transportation are easy.
(6) A production method of the fibrous membrane provided by the invention is easy in process, and short in production time, so that products can be effectively prevented from being contaminated in the processing, the product quality is easy to control, the product standards are easy to implement, and the low-cost and efficient industrialized production of products can be realized.

**Brief Description of the Drawings**

Figure 1 is a scanning electron microscope (SEM) picture of the section of a fluffy fibrous membrane obtained in Example 4;
Figure 2 is an SEM picture of the section of an electrospinning membrane obtained in step (2) of Example 4;

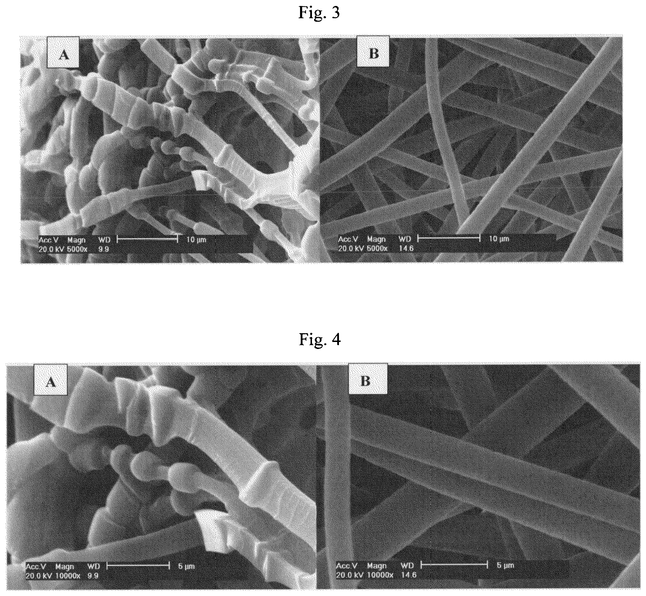

Figure 3 is a 5,000$\times$ SEM comparison picture of fibers of the fluffy fibrous membrane A obtained in Example 4 and the electrospinning membrane B obtained in step (2) of Example 4;
Figure 4 is a 10,000x SEM comparison picture of fibers of the fluffy fibrous membrane A obtained in Example 4 and the electrospinning membrane B obtained in step (2) of Example 4;
Figure 5 is a repair effect picture of a PP group in Example 8 one month after surgery;
Figure 6 is a repair effect picture of a PVDF1 group in Example 8 six months after surgery;
Figure 7 is a repair effect picture of a PVDF2 group in Example 8 six months after surgery;
Figure 8 is a repair effect picture of a PVDF2 group in Example 8 six months after surgery;
Figure 9 is a repair effect picture of a PCU group in Example 8 six months after surgery;
Figure 10 is a pathologic figure of a PLLA1 group in Example 10 three months after surgery;
Figure 11 is a pathologic figure of a PLLA2 group in Example 10 three months after surgery;
Figure 12 is a pathologic figure of an animal origin group in Example 10 three months after surgery;
Figure 13 is an anatomical figure of a PLLA1 group in Example 10 three months after surgery;
Figure 14 is an anatomical figure of a PLLA2 group in Example 10 three months after surgery;
Figure 15 is an anatomical figure of an animal origin group in Example 10 three months after surgery;
Figure 16 is an illustrative figure of a tension-free urinary incontinence sling of the invention;

Figure 17 is another illustrative figure of the tension-free urinary incontinence sling of the invention;

Figure 18 is an illustrative figure of a pelvic floor patch used for forepelvis of the invention;

Figure 19 is an illustrative figure of a pelvic floor patch used for posterior pelvis of the invention;

Figure 20 is an anatomical effect figure of a PVDF fluffy fibrous layer of a group IV in Example 21 four weeks after being implanted between gaps of vagina and bladder of a miniature pig;

Figure 21 is an effect figure of a PP mesh of a group I in Example 21 four weeks after being implanted between gaps of vagina and bladder of a miniature pig;

Figure 22 is an animal anatomical figure of an anti-adhesion tissue repair membrane (material No.1) prepared by combining Example 1 with Example 25;

Figure 23 is an animal anatomical figure of an anti-adhesion tissue repair membrane (material No.3) prepared by combining Example 23 with Example 25.

Description of the reference numerals

1    Main body part
2    End part
3    Outer contour
4    Through hole
5    Through hole in patch
6    Massive part in the middle of patch
7    Arm-like structure at the peripery of patch
8    Traction part of arm-like structure of patch

**Modes for Carrying out the Invention**

One aspect of the present invention relates to a fibrous membrane used for tissue repair and a preparation method thereof, and the other aspect of the present invention relates to applications of the fibrous membrane used for tissue repair in an implantable membrane used for tissue repair for treating female pelvic floor dysfunction and an anti-adhesion tissue repair membrane as a fluffy fibrous layer (A) as well as preparation methods thereof.

**I. Fibrous membrane used for tissue repair of the invention**

The fibrous membrane used for tissue repair of the invention is formed by interweaving fibrous filaments with diameters of 10nm-100$\mu$m, and has a porous structure, with the fluffiness of 200-2,000cm$^3$/g. The diameters of the fibrous filaments are 10nm-100$\mu$m. The fluffiness of the fibrous membrane used for tissue repair of the invention is preferably 600-1600cm$^3$/g, its softness is 50-500mN, more preferably 200-450 mN, its average pore size is 50-500$\mu$m, the thickness is 0.2-2mm and the tensile strength is preferably 20-80N/cm.

The fluffiness mentioned in the present invention refers to 1,000 times of a ratio of apparent thickness to surface density of the fibrous membrane, namely:

$$\text{Fluffiness } B = \text{Apparent thickness } T_0 / \text{Surface density } \omega \times 10^3$$

The fluffiness is represented by cm$^3$/g, the apparent thickness is represented by mm, and the surface density is represented by g/m$^2$ A testing method of the apparent thickness $T_0$ is carried out using an FAST-1 compressibility fabric style instrument according to GB/T 7689.1-2001, and the apparent thickness $T_0$ is expressed as the difference between the thickness (mm) of the fibrous membrane under the intensity of pressure of 2cN/cm$^2$ and its thickness (mm) under the intensity of pressure of 100cN/cm$^2$. According to a testing mode of the surface density $\omega$, under the condition of neglecting the thickness of the fibrous membrane, the weight per unit area of a single face is measured.

The softness of the present invention refers to the sum of a bending resistant force of the membrane tested according to a method in GB/T 8942-2002 and the maximum vector of a friction force between the membrane and a gap, expressed in mN. The smaller the value of the softness is, the softer the membrane will be.

The diameters of the fibrous filaments in the present invention are measured by means of an SEM; the average pore size is measured by a bubble point method using a capillary flow pore size analyzer with reference to ASTM D 6767-2002; the tensile strength is measured by a method in *Measurement for Fabric Breaking Strength and Elongation at Break* of GB/T3923.1-1997; and the thickness is measured by a compressibility fabric style instrument according to a method in GB/T 7689.1-2001.

<Preparation method of fibrous membrane used for tissue repair>

A preparation method of the fibrous membrane used for tissue repair of the invention comprises the step of electrostatic spinning.

A preferable preparation method of the fibrous membrane used for tissue repair of the invention can be carried out by combining an electrostatic spinning technology with a solvent dissolving method, specifically comprising the following steps:

(1) dissolving two fibrous filament materials of different solubility properties in corresponding solvents respectively to obtain two kinds of homogeneous fibrous filament material solutions;

(2) placing the two kinds of homogeneous fibrous filament material solutions obtained in step (1) into different electrostatic spinning injection syringes respectively, and uniformly arranging injection needles corresponding to the two fibrous filament materials on a high-voltage power panel for electrostatic spinning to obtain a fibrous membrane formed by two kinds of fibrous filaments of different solubility properties intricately intersecting;

(3) According to the solubility properties of the fibrous filament materials, selecting a suitable solvent to dissolve one kind of fibrous filaments in the fibrous membrane prepared in step (2), and keeping the other kind of fibrous filaments unchanged to obtain the fibrous membrane used for tissue repair.

In the above step (2), the rate of a micro-injection pump is preferably adjusted to be 0.1-15.0ml/h, more preferably 3-6ml/h.

In the above step (2), the voltage of a high-voltage generator is preferably adjusted to be 5-45KV, more preferably 20-36 KV.

In the above step (2), the receiving distance of a receiving device is preferably adjusted to be 5.0-30.0cm, more preferably 15.0-20.0cm.

In the invention, the two kinds of fibrous filament materials of different solubility properties refer to that only one of the two kinds of fibrous filament materials can be dissolved in a certain solvent. Preferably one kind of fibrous filament material is PCU (polycarbonate polyurethane) or PVDF (polyvinylidene fluoride). Preferably the other kind of fibrous filament material is PLLA (L-polylactic acid) or PLGA (polylactic-co-glycolic acid).

Another preferable preparation method of the fibrous membrane used for tissue repair of the invention comprises the following steps:

(1) dissolving a fibrous filament material in a solvent to obtain a homogeneous fibrous filament material solution;

(2) placing the homogeneous fibrous filament material solution obtained in step (1) into an electrostatic spinning injection syringe, carrying out electrostatic spinning to obtain fiber, and receiving the fiber into a membrane-like structure to obtain a fibrous membrane;

(3) pre-freezing the fibrous membrane prepared in step (2) after being subjected to ultrasonic swelling with a solvent at a predetermined temperature, and then carrying out vacuum freeze drying to obtain the fibrous membrane used for tissue repair.

In the above step (2), the rate of the micro-injection pump is preferably adjusted to be 0.1-15.0ml/h, more preferably 3-6ml/h.

In the above step (2), the voltage of the high-voltage generator is preferably adjusted to be 5-45KV, more preferably 30-36 KV.

In the above step (2), the receiving distance of the receiving device is preferably adjusted to be 5.0-30.0cm, more preferably 15.0-20.0cm.

In the above step (3), the fibrous membrane prepared in step (2) is preferably infiltrated with an ethanol aqueous solution with a concentration of 50%-95%, and then subjected to ultrasonic swelling.

In the above step (3), a solvent used for the ultrasonic swelling of the fibrous membrane is preferably water for injection.

In the above step (3), the fibrous membrane prepared in step (2) is preferably infiltrated in the ethanol aqueous solution with a concentration of 50%-95%, then placed into an ultrasonic machine containing the water for injection, subjected to the ultrasonic swelling for 5-15 minutes and then kept standing, the water for injection is replaced, the ultrasonic swelling is carried out again, and the operation is repeated like this till ethanol is completely replaced with the water for injection. Afterwards, the fibrous membrane in an ultrasonic swelling state with the water for injection is placed into a freeze drying oven for pre-freezing at -50 DEG C for 2-5 hours, and then vacuum is switched on for vacuum freeze drying for 20-26 hours to obtain the fibrous membrane used for tissue repair.

Another preferable preparation method of the fibrous membrane used for tissue repair of the invention comprises the following steps:

(1) dissolving a fibrous filament material in a solvent to obtain a homogeneous fibrous filament material solution;
(2) placing the homogeneous fibrous filament material solution obtained in step (1) into an electrostatic spinning injection syringe, carrying out electrostatic spinning to obtain fiber, and receiving the fiber into a membrane-like structure to obtain a fibrous membrane;
(3) stretching the fibrous membrane prepared in step (2) along the direction of horizontal axis or longitudinal axis of the fibrous membrane, and after stopping stretching, sizing the fibrous membrane in this stretching state; and then stretching the fibrous membrane along the direction perpendicular to the above stretching direction, and after stopping stretching, sizing the fibrous membrane in this stretching state to obtain the fibrous membrane used for tissue repair.

In the above step (2), the rate of the micro-injection pump is preferably adjusted to be 0.1-15.0ml/h, more preferably 3-6ml/h.

In the above step (2), the voltage of the high-voltage generator is preferably adjusted to be 5-45KV, more preferably 30-36 KV.

In the above step (2), the receiving distance of the receiving device is preferably adjusted to be 5.0-30.0cm, more preferably 15.0-20.0cm.

In the above step (3), the horizontal and longitudinal stretching rates are preferably 50-400mm/min respectively, more preferably 50 mm/min-200 mm/min.

In the above step (3), the horizontal and longitudinal stretching lengths are preferably 1.5-6.0 times of the original lengths respectively.

In the above step (3), preferably, two sides of the fibrous membrane prepared in the above step (2) are clamped with a fixture, the temperature is set to be 0-30 DEG C below a thermal deformation temperature of the material, the fibrous membrane is stretched at a uniform rate of 50-400mm/min, the stretching is not stopped until the length of the fibrous membrane is 1.5-6.0 times the original length, the fibrous membrane is sized in this stretching state for 1-4h at room temperature and then taken down, other two sides of the fibrous membrane are clamped with the fixture, the temperature is set to be 0-30 DEG C below the thermal deformation temperature of the material, the fibrous membrane is stretched at the uniform rate of 50-400mm/min along a direction perpendicular to the previous stretching direction, the stretching is not stopped until the length of the fibrous membrane is 1.5-6.0 times of the original length, and the fibrous membrane is sized in this stretching state for 1-4h at room temperature to obtain the fibrous membrane used for tissue repair.

The fibrous filaments used in the fibrous membrane used for tissue repair of the invention can be made from biodegradable materials, non-biodegradable materials or their combination, wherein the biodegradable materials can be synthetic biodegradable materials or natural polymeric materials; and the non-biodegradable materials are preferably fluoropolymer materials, polypropylene materials or polyurethane materials.

The biodegradable materials are preferably polylactic acid (PLA), especially L-polylactic acid (PLLA), polycaprolactone (PCL), polyglycolic acid (PGA), polylactic-co-glycolic acid (PLGA) or 1,3-propanediol polymer (PDO), collagen, gelatin, fibrin, silk-fibroin, elastin mimetic peptide polymer, chitosan and modified chitosan. The fluoropolymer materials are preferably polyvinylidene fluoride (PVDF) or polytetrafluoroethylene (PTFE).

The polypropylene materials are preferably syndiotactic polypropylene.

The polyurethane materials are preferably polyurethane (PU), polycarbonate polyurethane (PCU), polyether-based polyurethane or silane-modified polyurethane (SPU).

Other materials that may be used include: polyethylene glycol, polyethylene terephthalate (PET), polymethyl methacrylate, poly(3-hydroxybutyrate-co-3-hydroxyvalerate), poly(3-hydroxybutyrate-co-3-hydroxyhexanoate), polyphosphate, polyamino formic anhydride, polyesteramide, polyvinyl alcohol, poly(para-dioxanone), polycarbonate, alginate, chondroitin sulfate, heparin, glucosan, alginic acid, etc.

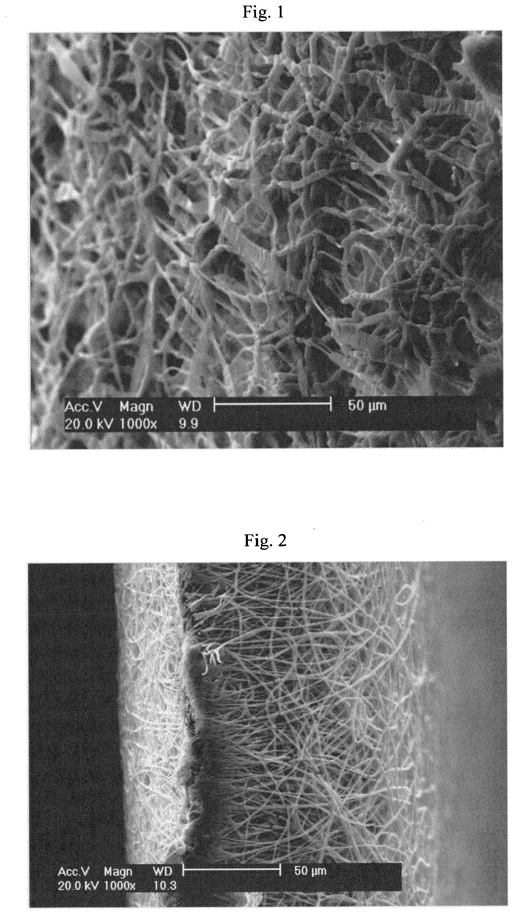

Figure 1 is a scanning electron microscope (SEM) picture of the section of the fibrous membrane used for tissue repair obtained according to the above method in Example 4 of the invention, and Figure 2 shows an electrospinning membrane obtained after step (2) according to the above method in the Example 4. As shown in Figure 1 and Figure 2, it can be seen, the fibrous membrane used for tissue repair of the invention is different from the electrospinning membrane obtained by common electrospinning in microscopic fiber structure, and in fluffiness and softness macroscopically, with the fluffiness and softness changing from $130cm^3/g$ and 870mN to $1,100cm^3/g$ and 400mN respectively, which means that the fibrous membrane used for tissue repair of the invention has better fluffiness and softness.

The fibrous membrane used for tissue repair of the invention can be further compounded with a woven layer to obtain a composite fibrous membrane used for tissue repair. The composite fibrous membrane used for tissue repair can be compounded with the woven layer by means of gomphosis, suture, adhesion, heat fusion, ultrasonic fusion, etc.

Further, an integrated structure of the fibrous membrane and the woven layer can also be formed by weaving or interpenetrating weaving lines taking the fibrous membrane used for tissue repair of the invention as a base material. The woven layer has better mechanical strength, can enhance the mechanical properties of the fibre membrane used for tissue repair of the invention after being combined with it, and is especially applicable to tissue repair applications with high requirements for mechanical strength, such as hernia repair, recovery of female pelvic floor prolapse and tendon repair; meanwhile, the fibrous membrane used for tissue repair of the invention is beneficial to rapid ingrowth of cells to have the effect of fixing the membrane, combined with its softness, displacement of the woven layer or damage caused by its friction to surrounding tissues can be reduced or avoided, and it is especially applicable to the treatment of female pelvic floor dysfunction.

The woven layer is made from synthetic polymeric materials, such as polypropylene, polyvinylidene fluoride, polytetrafluoroethylene, polyester, polylactic acid and PLGA, preferably a polypropylene woven membrane.

<Applications of fibre membrane used for tissue repair>

The present invention also provides applications of the fibre membrane used for tissue repair in the production of products for tissue repair. The term "tissue repair" used in the present invention includes not only regenerative and/or integral repair and recovery after tissue defect and/or loss, but also position and/or functional recovery after tissues and/or organs deviate from normal anatomical positions or normal physiological structures are changed (such as relaxation, prolapse, bulging or displacement, etc.). For example, the "tissue repair" of the invention includes but not limited to hernia repair, treatment of female pelvic floor dysfunction (including recovery of prolapse and bulging of pelvic cavity organs such as uterus, vagina, rectum, urethra and bladder and/or relaxation of muscular tissue of pelvic cavity or fistula repair), and tissue repair (filling) or structure recovery by using artificial tissue (such as artificial rotator cuff, dura (spinal dura mater), skin, pericardium, blood vessels, nerve conduits, periodontium, ligament, tendon, bones, etc.) materials (such as membranes, brackets, etc.). Preferably, the fibrous membrane used for tissue repair of the invention is used in the production of a hernia repair patch, a treatment system of female pelvic floor dysfunction (including a pelvic floor repair patch, a urinary incontinence sling or a fistula repair patch), an artificial rotator cuff, a dura (spinal dura mater) repair patch, artificial skin, a pericardium repair patch, artificial blood vessels, artificial nerve conduits, artificial periodontium, artificial ligament, artificial tendon, bone repair (filling) products, etc.

The present invention further provides products used for tissue repair prepared from the fibrous membrane used for tissue repair.

The products used for tissue repair of the present invention are preferably the hernia repair patch, the treatment system of female pelvic floor dysfunction (including the pelvic floor repair patch, the urinary incontinence sling or the fistula repair patch), the artificial rotator cuff, the dura (spinal dura mater) repair patch, the artificial skin, the pericardium repair patch, the artificial blood vessels, the artificial nerve conduits, the artificial periodontium, the artificial ligament, the artificial tendon, the bone repair (filling) products, etc.

The present invention further provides applications of the fibrous membrane used for tissue repair and the products used for tissue repair in tissue repair. Preferably, the present invention provides the applications of the fibrous membrane used for tissue repair and the products used for tissue repair in hernia repair, treatment of female pelvic floor dysfunction (including but not limited to pelvic floor repair, treatment of urinary incontinence or fistula repair), rotator cuff repair, dura (spinal dura mater) repair, skin tissue repair, pericardium repair, blood vessel repair, nerve conduit repair, periodontium repair, ligament repair, tendon repair and bone repair (filling).

**II. Implantable membrane used for tissue repair of the invention**

The present invention also relates to an implantable membrane used for tissue repair for treating female pelvic floor dysfunction. The implantable membrane comprises a fluffy fibrous layer (A), wherein the fluffy fibrous layer (A) is formed by interweaving fibrous filaments with diameters of 10nm-100$\mu$m, and has a porous structure, with the fluffiness of 400-1,500cm$^3$/g, and the softness of 50-500mN. The implantable membrane of the invention can be a single-layer membrane or a multi-layer membrane. When the implantable membrane is the single-layer membrane, only the fluffy fibrous layer (A) is included.

When the implantable membrane is the multi-layer membrane, it may comprise one or more fluffy fibrous layers (A), without special restrictions on materials, structures and preparation methods of other layers, and any materials, structures and preparation methods that can be used in the treatment of female pelvic floor dysfunction in the prior art can be used. Preferably, the implantable membrane can further comprise a directional fibrous layer (B), wherein the directional fibrous layer (B) is a layer with a porous three-dimensional structure formed by the directional arrangement of fibrous filaments. All layers of the implantable membrane can be combined by means of electrostatic spinning, ultrasonic fusion or suture, etc.

The above fibrous membrane used for tissue repair can be used as the fluffy fibrous layer (A) of the implantable membrane of the invention, which is preferably formed by interweaving fibrous filaments with diameters of 10nm-100$\mu$m, and has a porous structure, with the fluffiness of 400-1,500cm$^3$/g, and the softness of 50-500mN. The diameters of the fibrous filaments are preferably 500nm-5$\mu$m, the fluffiness is preferably 800-1,300cm$^3$/g, and the softness is preferably 200-450mN. Further, the average pore size of the fluffy fibrous layer (A) of the invention is 50-500$\mu$m, the thickness is 0.2-2mm, preferably 0.2-1.0mm, and the tensile strength is preferably 20-80N/cm.

The preparation method of the above fibrous membrane used for tissue repair can be adopted as a production method of the fluffy fibrous layer (A) of the implantable membrane of the invention.

<Directional fibrous layer (B)>

The directional fibrous layer (B) is a layer with a porous three-dimensional structure formed by the directional arrangement of fibrous filaments with diameters of 10nm-20$\mu$m. It has regular fiber orientation, and can simulate the orientation of collagen fibers in extracellular matrix in fascia, guide cell growth, provide a directional skeleton for cells and newly secreted extracellular matrix, and structurally simulate tissue structure of the body itself as much as possible, thereby being very conducive to ingrowth of fibroblasts and capillaries, playing a key role in self-repair of muscles and fascia, and achieving long-term stable repair effect. Therefore, better postoperative repair effect can be achieved, and the recurrence rate can be reduced.

In the present invention, the directional fibrous layer (B) is preferably prepared by using electrostatic spinning. The operating conditions are as follows: the rate of the micro-injection pump is adjusted to be 0.1-15.0ml/h, the voltage of the high-voltage generator is adjusted to be 5-45KV, the receiving distance of the receiving device is adjusted to be 5.0-30.0cm, the movement speed of an electrospinning needle is adjusted to be 1-20cm/s, and the rotating speed of a receiving roller is 2,000-6,000 revolutions/min.

<Fibrous material>

The implantable membrane of the invention can be made from non-biodegradable materials, biodegradable materials or their combination. The non-biodegradable materials mainly include fluoropolymer materials, such as PVDF and PTFE; polyolefins, such as polyethylene and polypropylene; and polyurethane materials, such as PU, PCU, polyether-based polyurethane and SPU. The biodegradable materials mainly include synthetic materials such as PLA, PCL, PGA, PLGA and PDO; natural polymeric materials such as collagen, gelatin, fibrin, silk-fibroin and elastin mimetic peptide polymer; and chitosan and modified chitosan. The fluffy fibrous layer (A), the directional fibrous layer (B) and other layers can be made from same or different fibrous materials, wherein the directional fibrous layer (B) is preferably prepared from the PVDF material that has excellent tissue compatibility and durability, as well as good mechanical strength; and the fluffy fibrous layer (A) is preferably prepared from PVDF, PCU and PLA.

Other materials that may be used also include: polyethylene glycol, polyethylene terephthalate (PET), polymethyl methacrylate, poly(3-hydroxybutyrate-co-3-hydroxyvalerate), poly(3-hydroxybutyrate-co-3-hydroxyhexanoate), polyphosphate, polyamino formic anhydride, polyesteramide, polyvinyl alcohol, poly(para-dioxanone), polycarbonate, alginate, chondroitin sulfate, heparin, glucosan, alginic acid, etc.

<Other layers>

The implantable membrane of the invention can also comprise other layers except for the fluffy fibrous layer (A) and the directional fibrous layer (B), which can be a non-oriented fibrous layer (C), a woven mesh layer (D) or a memory metal layer (E) prepared by a common electrospinning technology.

The implantable membrane of the invention can also comprise a layer containing anti-infective drugs, hemostatic drugs and/or micro-nano particles, wherein the anti-infective drugs, the hemostatic drugs and/or the micro-nano particles can be positioned in the fluffy fibrous layer (A), the directional fibrous layer (B) and the non-oriented fibrous layer (C), also can be positioned in the other layers except for the above layers, and also can be adsorbed on the surface of the implantable membrane.

The anti-infective drugs include ampicillin, spiramycin, sulfa, quinolone and/or cephalosporin antibiotics. The hemostatic drugs include 6-aminocaproic acid, p-aminomethyl benzoic acid, tranexamic acid, radix notoginseng and/or Yunnan Baiyao powder. The micro-nano particles include $SiO_2$, $TiO_2$, ZnO, Ag, Ni, quaternary ammonium salt, chitosan, calcium alginate, polyvinyl alcohol and/or natural macromolecular nanoparticles.

<Post-treatment>

The implantable membrane of the invention can be further modified through post-treatment, for example, it can be treated through one or more ways of drilling, heating and soaking. The fibrous membrane can also be sutured by using metal wires or polymer fibers.

During the drilling treatment, methods such as stamping drilling, laser drilling and local pressure melting drilling can be used to form a through hole penetrating through the upper and lower surfaces of the fibrous membrane, wherein the laser drilling is preferable. By adjusting and optimizing parameters, after the hole is formed through laser cutting, a part of the periphery of the hole can be molten at high temperature instantly to become a dense structure, and the width of the molten part at the periphery of the hole is about 0.02-0.05mm. The molten part can play a role in fixing the pore size, and maintaining overall mechanical properties of the product. In the preparation of an implantable patch using electrostatic spinning, a receiving plate with metal and insulating staggered grids or with patterning electrostatic repulsion grains can be adopted for drilling.

The long-term study of a pelvic floor repair system shows that, the pore size of the implantable membrane is crucial to the treatment effect. However, a stent prepared by a conventional electrospinning process is high in porosity, but very small in pore size; and a general pore-forming method will greatly influence the mechanical properties of the electrospinning stent. In the present invention, through research and comparison of different post-treatment technologies, and through a large number of in vitro and in vivo biological experiments, a membrane material with high mechanical strength, more softness and better treatment effect is obtained after repeated optimization, which has appropriate pore size and pore arrangement. The adoption of staggered arrangement of pores with certain sizes or combination of pores with different sizes can be more conducive to tissue ingrowth, and quick discharge of metabolites during tissue and cell growth while enhancing the exchange of internal and external materials. Meanwhile, the pores provide more space for tissue growth, thereby being more conducive to capillary penetration.

In order to maintain the mechanical strength, a combination of large pores and small pores can be adopted for preparation: large pores with diameters of 0.8-1.6mm are drilled at intervals being 0.5-1cm; and 2-4 small pores with diameters of 0.4-0.6mm are uniformly drilled among every four large pores. Therefore, the number of pores can be increased while maintaining the mechanical strength of the repair patch, thus being more conducive to rapid through growth of tissues.

<Implantable medical device>

An implantable medical device for treating female pelvic floor dysfunction of the invention comprises the implantable membrane of the invention. Particularly, the implantable medical device can be a tension-free urinary incontinence sling or a pelvic floor repair patch.

The tension-free urinary incontinence sling (hereinafter referred to as the sling) comprises a main body part and an end part, wherein the main body part is made from the implantable membrane of the invention, and the end part is used for surgical instrument traction and/or fixation.

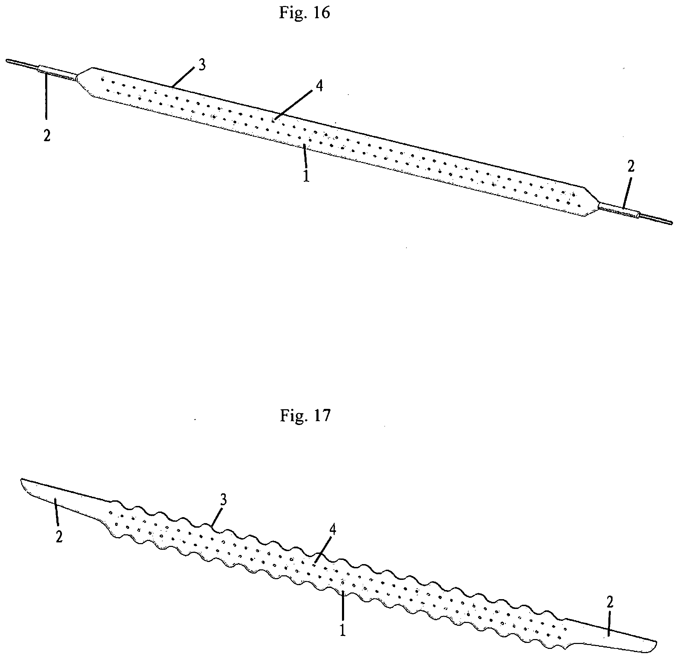

Exemplarily, the sling of the invention can have the appearance as shown in Figures 16-17. It comprises the main body part 1 and the end part 2. After the sling is implanted into a human body, the main body part 1 contacts with the anterior wall of vagina and an interlayer of urethra, and is embedded into obturator fascia to enable it to keep a tensioning state so as to exert an upward supporting force on the urethra, and the end part 2 is used for surgical instrument traction and/or fixation. The width of the sling is preferably 5mm-30mm, and its thickness is preferably 0.1mm-2mm. The main body part of the sling can have a linear (as shown in Figure 16) or corrugated (as shown in Figure 17) outer contour 3, wherein the corrugated concave-convex width difference is preferably 1mm-5mm. In a specific embodiment, the end part can also have a corrugated outer contour which is the same as or similar to that of the main body part. The sling with the corrugated outer contour is preferably used, so that the sling can be fixed more firmly in an obturator membrane of pelvis. Preferably, the main body part 1 of the sling is provided with a through hole 4 with a diameter of 0.1mm-3mm penetrating through the upper and lower surfaces of the main body part of the sling to be advantageous to tissue ingrowth from the pore, and play a role in further anchoring a mesh. More preferably, the diameter of the through hole 4 is 1.2mm-3mm.

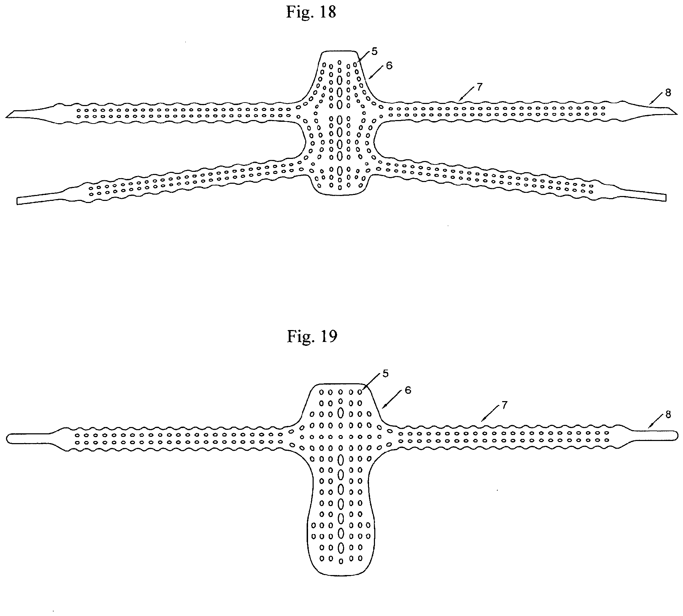

A pelvic floor patch comprises a central main body part used for repairing prolapse and bulging of pelvic organs (such as bladder, uterus, rectum, vagina and small intestine), wherein the central main body part is made from the implantable membrane of the invention. The implantable membrane used in the pelvic floor patch preferably comprises the directional fibrous layer (B). Exemplarily, the pelvic floor patch of the invention can be in a shape as shown in Figure 18 (used for forepelvis) and Figure 19 (used for posterior pelvis). It comprises a central massive part 6 used for suspending a procident pelvic organ and an arm-like structure 7 at the periphery, wherein the tail end of the arm-like structure 7 is provided with a traction part 8 of the arm-like structure, and preferably the central massive part 6 and the peripheral arm-like structure 7 of the patch are provided with through holes 5 penetrating through the upper and lower surfaces. The central massive part 6 can be adjusted according to the shape of the pelvic organ to be repaired, and the shape and number of the arm-like structure 7 can also be specifically adjusted based on the visceral organ to be repaired.

The implantable medical device made from the implantable membrane of the invention can be used for treating female pelvic floor dysfunction. The tension-free urinary incontinence sling of the invention can be used for treating female urinary incontinence, and the pelvic floor patch can be used for repairing prolapse and bulging of pelvic organs.

**III. Anti-adhesion tissue repair membrane of the invention**

The surface layer on one side of the anti-adhesion tissue repair membrane of the invention is a fluffy fibrous layer (A'), and the surface layer on the other side is an anti-adhesion layer (B'); and the fluffy fibrous layer (A') is formed by interweaving fibrous filaments with diameters of 10nm-100$\mu$m, and has a porous structure, with the fluffiness of 200-2,000cm$^3$/g, and the softness of 50-500mN.

The anti-adhesion tissue repair membrane of the invention has at least the above two-layer structure, wherein an intermediate layer can be optionally arranged between the fluffy fibrous layer (A') and the anti-adhesion layer (B'), without special restrictions on materials, structures and preparation methods of the intermediate layer, and any materials, structures and preparation methods which can be used for tissue repair in the prior art may be used.

<Fluffy fibrous layer (A')>

The fluffy fibrous layer (A') of the anti-adhesion tissue repair membrane of the invention adopts the above fibrous membrane used for tissue repair of the invention, and can be prepared by a method the same as the preparation method of the fibrous membrane.

The fluffy fibrous layer (A') of the anti-adhesion tissue repair membrane of the invention is formed by interweaving fibrous filaments with diameters of 10nm-100$\mu$m, and has a porous structure, with the fluffiness of 200-2,000cm$^3$/g, and the softness of 50-500mN. The diameters of the fibrous filaments are preferably 500nm-5$\mu$m, and the fluffiness is preferably 400-1,500cm$^3$/g, more preferably 800-1,300cm$^3$/g; and the softness is preferably 200-450mN. Further, the average pore size of the fluffy fibrous layer (A') of the invention is 50-500$\mu$m, the thickness is 0.2-2mm, preferably 0.2-1.0mm, and the tensile strength is preferably 20-80N/cm.

<Fibrous materials>

The fluffy fibrous layer (A') and the intermediate layer of the invention can be made from the fibrous materials including non-biodegradable materials, biodegradable materials or their combination. The non-biodegradable materials mainly include fluoropolymer materials, such as PVDF and PTFE; polyolefins, such as polyethylene and polypropylene; and polyurethane materials, such as PU, PCU, polyether-based polyurethane and SPU. The biodegradable materials mainly include synthetic materials such as PLA, PCL, PGA, PLGA and PDO; natural polymeric materials such as collagen, gelatin, fibrin, silk-fibroin and elastin mimetic peptide polymer; and chitosan and modified chitosan. The fluffy fibrous layer (A'), the fibrous layer (D) and other layers can be made from same or different fibrous materials, wherein the fluffy fibrous layer (A') is preferably prepared from PVDF, PCU and PLA.

Other materials that may be used also include: polyethylene glycol, polyethylene terephthalate (PET), polymethyl methacrylate, poly(3-hydroxybutyrate-co-3-hydroxyvalerate), poly(3-hydroxybutyrate-co-3-hydroxyhexanoate), polyphosphate, polyamino formic anhydride, polyesteramide, polyvinyl alcohol, poly(para-dioxanone), polycarbonate, alginate, chondroitin sulfate, heparin, glucosan, alginic acid, etc.

<Anti-adhesion layer (B')>

The anti-adhesion layer (B') of the invention is combined with other layers by means of spraying or ultrasonic welding. When the anti-adhesion tissue repair membrane of the invention is a double-layer membrane, the anti-adhesion layer (B) is preferably compounded with the fluffy fibrous layer (A') by means of spraying. Through this manner, the anti-adhesion layer (B') can infiltrate into the fluffy fibrous layer (A') to be tightly combined with it.

The anti-adhesion layer obtained by spraying has certain micropores, so as to have the anti-adhesion effect while guaranteeing the supply of nutrients. In a spraying process, a solution is squeezed out through hydraulic pressure, and atomized at a nozzle to form some tiny droplets which are connected with one another after contacting with the fluffy fibrous layer (A') and infiltrating into surface fibers, so that a part of pores formed in the fibers are closed, and the micropores formed by the droplets can be controlled by controlling the sizes and spacing of the droplets. The diameters of the micropores obtained by this method can be within the range of 0.05-3$\mu$m. The anti-adhesion layer (B') obtained by spraying has certain softness and compliance, so that it can produce corresponding deformation under the condition that the fluffy fibrous layer (A') is stressed, so as to ensure the tight combination of them under stress.

Spraying fluid for the anti-adhesion layer (B') is preferably prepared by using a chitosan and/or carboxymethyl chitosan solution. The molecular weight of the chitosan and/or carboxymethyl chitosan is preferably 300,000-1,000,000, more preferably 500,000-800,000, and its degradation time is about 1-3 months. A solvent for the chitosan and/or carboxymethyl chitosan solution can be an acetic acid aqueous solution, hydrochloric acid, dilute sulphuric acid, dilute nitric acid, purified water, etc. The solvent is preferably the acetic acid aqueous solution, wherein the content of acetic acid is 1%-36% (mass fraction), preferably 3%-15%. The concentration of chitosan and/or carboxymethyl chitosan in the acetic acid aqueous solution is 1%-15% (mass/volume fraction), preferably 3%-8%.

The spraying fluid for spraying the anti-adhesion layer (B') does not have special restrictions on an adopted spraying device. But the spraying device needs to be sealed, and can enable feed liquid loaded therein to be pressed by means of pressurizing so as to be sprayed out from a nozzle of the spraying device.

The spraying method described below is preferably used in the present invention, which includes the following steps:

(1) dissolving chitosan and/or carboxymethyl chitosan in a solution to form anti-adhesion layer spraying fluid;
(2) placing the anti-adhesion layer spraying fluid into a spraying device connected with a hydraulic press, setting the range of spraying parameters as follows: spraying flow, 0.005ml/min-0.1ml/min; scanning spacing, 10mm-30mm; and scanning speed, 1mm/s-50mm/s; spraying the anti-adhesion layer spraying fluid on one surface of the fluffy fibrous layer (A'), the woven layer (C), the fibrous layer (D) or other intermediate layers;
(3) post-treatment: soaking and drying, soaking a sample obtained in step (2) in an aqueous solution, removing residual solvent and then placing into an air dry oven for drying, with the drying temperature set to be 35-60 DEG C, and the treatment time of 2 days.

After the above spraying and post-treatment, cutting, packaging, EO sterilization and other steps can be carried out according to needs.

<Intermediate layer>

In the anti-adhesion tissue repair membrane of the invention, the intermediate layer can be arranged between the fluffy fibrous layer (A') and the anti-adhesion layer (B'), which can be the woven layer (C'), the fibrous layer (D') or other layers that can be used in the tissue repair membrane, such as a drug layer.

The anti-adhesion tissue repair membrane of the invention can have three-layer, four-layer or multi-layer structures. The first surface layer is the fluffy fibrous layer (A'), which is a fibrous layer with a specific fluffy structure, and also a fibrous membrane capable of rapidly promoting tissue repair and regeneration. The fibrous membrane is formed by interweaving fibrous filaments with diameters of 10nm-100$\mu$m, and has a porous structure, with the fluffiness of 200-2,000cm$^3$/g. The intermediate layer can be the woven layer (C'), or the fibrous layer (D') or a combination thereof, wherein the woven layer (C') is a woven mesh used for enhancing the strength of the overall patch; and the fibrous layer (D') can be a fibrous layer obtained by electrospinning, preferably a fibrous layer with a fluffy structure the same as the structure of the first layer. The surface layer on the other side is the anti-adhesion layer (B') made from a material having an anti-adhesion function. The fibrous layer with the fluffy structure and the woven mesh layer can be combined together by means of ultrasonic spot welding, suture, etc. The anti-adhesion layer and the intermediate layer can be combined together by means of spraying, ultrasonic welding, etc., preferably spraying.

According to the woven layer (C') of the invention, the woven mesh (which can be made from PP, PVDF, PTFE, PET, etc.) is preferably completely soaked in a polymer solution with certain concentration. The polymer material is preferably consistent with the fibrous material of the fluffy fibrous layer (A). After being soaked for a certain period of time, the woven mesh is taken out and oven dried by heating to enable fibrous filaments on the woven mesh to be wrapped with a relatively thin layer of polymer material.

The above anti-adhesion tissue repair membrane with the multi-layer structure is preferably used for repairing abdominal hernia and incisional hernia. This design is adopted for the following main reasons: (1) as the repair for the abdominal hernia and incisional hernia has higher requirements for mechanical strength, a common simple electrospinning membrane is difficult to meet the requirements, therefore the woven mesh is additionally arranged to ensure the mechanical strength; (2) for the hernia repair by using the simple woven mesh, repaired and grown tissues cannot be well combined with the woven mesh, so that the woven mesh cannot be fixed, and is easy to form erosion and shrinkage. Hence, two sides of the woven mesh are respectively and additionally provided with a layer of fibrous membrane with the specific fluffy structure, so as to enable the tissues to be well repaired and wrap the woven mesh to avoid its erosion and shrinkage; and (3) in the repair of the abdominal hernia and incisional hernia, the patch is required to have the anti-adhesion function for intraperitoneal repair, in order to prevent intestinal obstruction, necrosis and the like caused by adhesion of new tissue to intra-abdominal organs, so that a fourth-layer material with the anti-adhesion function is designed for the patch.

The anti-adhesion tissue repair membrane with the four-layer structure of the invention can be prepared by adopting the following method:

(1) preparing two fibrous membranes with specific fluffy structures and specific thickness according to the aforementioned preparation method of the fluffy fibrous layer (A');

(2) completely soaking a woven mesh (which can be made from PP, PVDF, PTFE, PET, etc.) meeting the requirements for the mechanical strength in a polymer solution with certain concentration, wherein the polymer material should be consistent with the material of the fibrous membranes in step (1); and after soaking for a certain period of time, taking out the woven mesh and oven drying by heating to enable fibrous filaments on the woven mesh to be wrapped with a relatively thin layer of polymer material;

(3) placing the woven mesh wrapped with the polymer obtained in step (2) between the two fibrous membranes in step (1), then placing on an ultrasonic welding machine together, and under certain ultrasonic power and pressure, welding the two fibrous membranes on two sides of the woven mesh;

(4) spraying the anti-adhesion material on one surface of the membrane prepared in step (3) by adopting the aforementioned spraying technology.

Examples

**<Preparation of fibrous membrane used for tissue repair: Examples 1-6>**

Example 1

(1) The PCU is dissolved in a mixed solvent of N,N-dimethyl formamide and tetrahydrofuran, the concentration of the PCU in the solution is adjusted to be 12g/100mL, and the mixing ratio (volume ratio) of the N,N-dimethyl formamide to the tetrahydrofuran is adjusted to be 1:1 to obtain a homogeneous fibrous filament material solution.

The PLLA is dissolved in an hexafluoro-iso-propanol (HFIP) solution, and the concentration of the PLLA in the solution is adjusted to be 5g/100mL to obtain a homogeneous fibrous filament material solution.

(2) The above two kinds of homogeneous fibrous filament material solutions are respectively put into two electrostatic spinning injection syringes, injection needles corresponding to the two materials are uniformly arranged on a high-voltage power panel, the rate of a micro-injection pump is adjusted to be 6ml/h, the voltage of a high-voltage generator is adjusted to be 22KV, the receiving distance of a receiving device is adjusted to be 20cm, a fibrous membrane formed by two kinds of fibrous filament materials with different solubility properties intricately intersecting is prepared through simultaneous electrostatic cospinning of the two materials, and electrostatic spinning is turned off after the fibrous membrane is 0.5mm thick.

(3) The membrane which is taken down is put into an HFIP solvent for ultrasonic swelling and dissolving for 6h to enable the PLLA material to be dissolved completely, while the PCU material remains unchanged, and the insoluble material is taken out from the solvent to obtain the fibrous membrane used for tissue repair.

The average diameter of fibers of the fibrous membrane used for tissue repair obtained in Example 1 is $2\mu m$, the membrane thickness is 0.5mm, the average pore size is $350\mu m$, the tensile strength is 25N/cm, the fluffiness is $970cm^3/g$, and the softness is 250mN.

Example 2

(1) The PVDF is dissolved in a mixed solvent of N,N-dimethyl formamide/acetone with the volume ratio of 4:6, and the concentration of the PVDF in the solution is adjusted to be 18g/100mL; and the PLLA is dissolved in an HFIP solution, and the concentration of the PLLA in the solution is adjusted to be 5g/100mL to obtain a homogeneous fibrous filament material solution.

(2) The above two kinds of homogeneous fibrous filament material solutions are respectively put into two electrostatic spinning injection syringes, the rate of a micro-injection pump is adjusted to be 5ml/h, in addition, electrospinning is carried out by adopting a method the same as the step (2) in the Example 1.

(3) The membrane which is taken down is put into an HFIP solvent for ultrasonic swelling and dissolving for 6h to enable the PLLA material to be dissolved completely to prepare the fibrous membrane used for tissue repair.

The average diameter of fibers of the fibrous membrane used for tissue repair is $3\mu m$, the membrane thickness is 0.5mm, the average pore size is $450\mu m$, the tensile strength is 35N/cm, the fluffiness is $1,640cm^3/g$, and the softness is 400mN.

Example 3

(1) The PVDF material is dissolved in a mixed solvent of N,N-dimethyl formamide/acetone with the volume ratio of 4:6, and the concentration of the PVDF in the solution is adjusted to be 18g/100mL to prepare a homogeneous fibrous filament material solution.

(2) The fibrous filament material solution obtained in step (1) is put into an electrostatic spinning injection syringe, the rate of a micro-injection pump is adjusted to be 6ml/h, the voltage of a high-voltage generator is adjusted to be 30KV, the receiving distance of a receiving device is adjusted to be 20cm, fibers are obtained by electrostatic spinning and received into a membrane-like structure, and the electrostatic spinning is turned off after the membrane thickness is about 0.5mm to obtain a fibrous membrane.

The average diameter of fibers of the fibrous membrane obtained herein is $2\mu$m, the membrane thickness is 0.5mm, the average pore size is 190$\mu$m, the tensile strength is 43N/cm, the fluffiness is 170cm$^3$/g, and the softness is 930mN.

(3) The fibrous membrane prepared in step (2) is completely infiltrated in an ethanol solution with the concentration of 95% by volume, then the fibrous membrane infiltrated by the ethanol solution is taken out and placed into an ultrasonic container containing water for injection to enable the fibrous membrane to be soaked in the water for injection completely, ultrasound is turned on and lasts for 10min under the power of 90W, after standing for 5-10min, the water for injection in the ultrasonic container is replaced, the ultrasound is turned on again and lasts for 10min under the power of 90W, and the operation is repeated for 7-8 times till ethanol in the solution is replaced completely. Afterwards, the fibrous membrane after being subjected to ultrasonic swelling with the water for injection is taken out and put into a freeze drying oven at -50 DEG C for pre-freezing for 4h, then vacuum freeze drying is turned on, and the pre-frozen fibrous membrane is subjected to vacuum freeze drying for 24h to obtain the fibrous membrane used for tissue repair.

The average diameter of fibers of the fibrous membrane used for tissue repair is $2\mu$m, the membrane thickness is 0.6mm, the average pore size is 400$\mu$m, the tensile strength is 48N/cm, the fluffiness is 1,530cm$^3$/g, and the softness is 420mN.

Example 4

(1) The PLLA material is dissolved in an HFIP solvent, and the concentration of the PLLA in the solution is adjusted to be 6g/100mL to prepare a homogeneous fibrous filament material solution.

(2) The fibrous filament material solution prepared in step (1) is put into an electrostatic spinning injection syringe, the voltage of a high-voltage generator is adjusted to be 20KV, the receiving distance of a receiving device is adjusted to be 15cm, in addition, electrospinning is carried out according to a method the same as the step (2) in the Example 3 to obtain a fibrous membrane.

The average diameter of fibers of the fibrous membrane obtained herein is $2\mu$m, the membrane thickness is 0.5mm, the average pore size is 115$\mu$m, the tensile strength is 33N/cm, the fluffiness is 130cm$^3$/g, and the softness is 870mN.

(3) Afterwards, two sides of the fibrous membrane are clamped with a fixture, and stretched at a uniform rate of 100mm/min under the condition that the temperature is 60 DEG C, the stretching is not stopped until the length of the fibrous membrane is 3 times of the original length, the fibrous membrane is sized in this stretching state for 4h at room temperature and then taken down, other two sides of the fibrous membrane are clamped with the fixture, and stretched at the uniform rate of 100mm/min under the condition that the temperature is 60 DEG C along a direction perpendicular to the previous stretching direction, the stretching is not stopped until the length of the fibrous membrane is 3 times of the original length, and the fibrous membrane is sized in this stretching state for 4h at room temperature to obtain the fibrous membrane used for tissue repair.

The average diameter of fibers of the fibrous membrane used for tissue repair is $2\mu$m, the membrane thickness is 0.6mm, the average pore size is 450$\mu$m, the tensile strength is 50N/cm, the fluffiness is 1,100cm$^3$/g, and the softness is 400mN.

Example 5

(1) The PVDF material is dissolved in a mixed solvent of N,N-dimethyl formamide/acetone with the volume ratio of 4:6, and the concentration of the PVDF in the solution is adjusted to be 18g/100mL to prepare a homogeneous fibrous filament material solution.

(2) The fibrous filament material solution obtained in step (1) is put into an electrostatic spinning injection syringe, and electrospinning is carried out according to a method the same as the step (2) in the Example 3 to obtain a fibrous membrane.

The average diameter of fibers of the fibrous membrane obtained herein is $2\mu$m, the membrane thickness is 0.5mm, the average pore size is 190$\mu$m, the tensile strength is 43N/cm, the fluffiness is 170cm$^3$/g, and the softness is 930mN.

(3) The fibrous membrane used for tissue repair is obtained by stretching according to a method the same as the step (3) in the Example 4, except for the fibrous membrane is stretched till its length is 2.5 times of the original length at the temperature of 95 DEG C.

The average diameter of fibers of the fibrous membrane used for tissue repair is $2\mu$m, the membrane thickness is 0.6mm, the average pore size is 400$\mu$m, the tensile strength is 65N/cm, the fluffiness is 1,300cm$^3$/g, and the softness is 450mN.

Example 6

(1) The PCU material is dissolved in a mixed solvent of N,N-dimethyl formamide/tetrahydrofuran with the volume ratio of 1:1, and the concentration of the PCU in the solution is adjusted to be 12g/100mL to prepare a homogeneous fibrous filament material solution.

(2) The fibrous filament material solution obtained in step (1) is put into an electrostatic spinning injection syringe, the rate of a micro-injection pump is adjusted to be 5ml/h, the voltage of a high-voltage generator is adjusted to be 36KV, the receiving distance of a receiving device is adjusted to be 25cm, fibers are obtained by electrostatic spinning and received into a membrane-like structure, and the electrostatic spinning is turned off after the membrane thickness is about 0.5mm to obtain a fibrous membrane.

The average diameter of fibers of the fibrous membrane obtained herein is $2\mu$m, the membrane thickness is 0.5mm, the average pore size is 160$\mu$m, the tensile strength is 52N/cm, the fluffiness is 110cm$^3$/g, and the softness is 510mN.

(3) The fibrous membrane used for tissue repair is obtained by stretching according to a method the same as the step (3) in the Example 4, except for the fibrous membrane is stretched till its length is 2.0 times of the original length at the temperature of 80 DEG C and sized for 6h.

The average diameter of fibers of the fibrous membrane used for tissue repair is $2\mu$m, the membrane thickness is 0.5mm, the average pore size is 260$\mu$m, the tensile strength is 60N/cm, the fluffiness is 230cm$^3$/g, and the softness is 470mN.

Example 7

The fluffy fibrous membranes prepared in the Examples 1, 2, 3, 4, 5 and 6 are respectively superposed with woven PP (3DMAX™ Mesh, a sample provided by the Second Affiliated Hospital of Sun Yat-Sen University) membranes, then 20,000Hz frequency ultrasounds (Futan Mechanical Equipment Co., Ltd., type: JT-200-S) are used, and all the above layers are connected together by means of point ultrasonic fusion every 10cm to obtain composite membranes of the fibrous membranes used for tissue repair and PP woven membranes respectively.

**<Animal experiments of fibrous membranes used for tissue repair: Examples 8-12>**

Example 8 Application of fibrous membranes in hernia repair

The fibrous membranes used for tissue repair prepared in the Examples 1 and 5, the fibrous membrane prepared in step (2) of the Example 5, and the clinically used PP mesh (3DMAX™ Mesh, the sample provided by the Second Affiliated Hospital of Sun Yat-Sen University) are cut into 3.5cm x 6cm pieces, cleaned, sterilized and made into a PCU hernia repair patch (made from the fibrous membrane of the Example 1), a PVDF1 hernia repair patch (made from the fibrous membrane of the Example 5), a PVDF2 hernia repair patch (made from the fibrous membrane obtained in step (2) of the Example 5) and a PP hernia repair patch (made from the PP mesh) respectively.

New Zealand rabbit experiments are carried out by using the above hernia repair patches. There are a total of 60 New Zealand rabbits, weighing 2.5-2.8Kg each, and aged 6-12 months. These experimental rabbits are randomly divided into four groups, namely a PCU group, a PVDF1 group, a PVDF2 group and a PP group respectively, with 15 experimental animals in each group. The animal in the experiment is anesthetized and subjected to skin preparation, lies on its back and is fixed on a board; after a surgical drape is disinfected, the skin at the center of the abdomen is incised by using a scalpel No. 4 along a white line on the rabbit's abdomen to form an incision that is about 8cm long and expose muscles, the full-thickness abdominal wall with the size of 2cm x 5cm (including peritoneum, muscles and fascia tissues) is resected; the PCU group, the PVDF1 group, the PVDF2 group and the PP group respectively adopt the PCU hernia repair patch, the PVDF1 hernia repair patch, the PVDF2 hernia repair patch and the PP hernia repair patch, and silk threads No.0 are used for interrupted suture between the patches and surrounding muscles while silk threads No.4 are used for interrupted suture between skin.

Postoperative routine observation and feeding for the animals are carried out. During the observation period, all the animals in the PCU group, the PVDF1 group and the PVDF2 group can take food and drink water normally, and the incisions are healed well, without infection or formation of abdominal hernia. The incisions of the five animals in the PP group bulge 5-7 days after surgery, three animals die 7-10 days after surgery, and it is found that severe adhesion is formed between the patches and viscera during anatomy. All the rest of the animals survive to the sampling time.

One month after surgery, five animals in each of the PCU group, the PVDF1 group and the PVDF2 group are dissected, while four animals in the PP group are dissected. Surgical sites of the animals in the PCU group and the PVDF1 group are soft in touch, while those of the animals in the PVDF2 group are slightly hard in touch compared with the PVDF1 group. The muscle layers are incised 2cm above the edges of the patches, the patches of the PCU group, the PVDF1 group and the PVDF2 group are not adhered to the viscera visually, blood capillaries grow into the center along the edges of the patches, and new tissues have basically covered the entire patches. Microscopic results show that a lot of hyperplasia of fibroblasts and collagen fibers, little lymphocyte infiltration ($\leq$25/HPF) and more blood capillary hyperplasia (5-10/HPF) are visible around the patches of the PCU group, the PVDF1 group and the PVDF2 group. And microscopically, there are more blood capillaries in the PCU group and the PVDF1 group compared with the PVDF2 group, and the difference is statistically significant ($P<0.05$). Thus it can be seen, the patches of the PCU group, the PVDF1 group and the PVDF2 group have better histocompatibility, wherein the higher fluffiness of the patches of the PCU group and the PVDF1 group is more advantageous to tissue ingrowth.

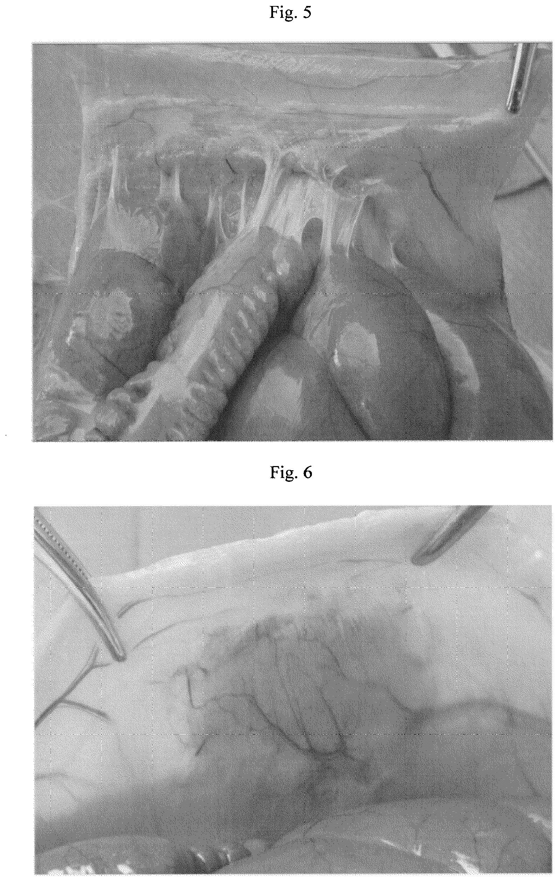

The surgical sites of the PP group are harder in touch than other three groups, and the presence of foreign bodies can be obviously felt. After incision of skin, it can be seen, a thin layer of new tissue covers the surface of each patch, but is easy to be torn from the patch. The muscle layers are incised 2cm around the patches, and the patches of the PP group are seriously adhered to viscera visually, and are not easy to separate (Figure 5). Microscopic results show that there are more holes inside the patches of the PP group, a small quantity of fibroblasts and collagen are formed around the holes, and a small amount of blood capillary hyperplasia ($\leq$2/HPF), and a large amount of infiltration of foreign body giant cells and lymphocytes are visible. Thus it can be seen, the patches of the PP group have poorer histocompatibility, and are not conducive to cell adhesion and obvious in rejection and immune inflammatory response.

Three months after surgery, four animals in the PP group are dissected, and five animals in each of the other groups are dissected. The patches and tissues within the range of 2cm around the patches are incised. The surgical sites of the PCU group and the PVDF1 group are soft in touch, and close to autologous tissues. The patches and skin are clear in layers, and easy to be separated from each other. No adhesion exists between the patches and visceral organs, and a large number of new tissues grow into the patches. Microscopic results show that the peripheries of the patches are tightly combined with collagen fibers and fibrocytes; and a large number of collagen fibers are visible inside the patches, with a small number of blood capillaries (3-5/HPF). The surgical sites of the materials in the PVDF2 group are softer in touch, the patches and the skin are clear in layers, and easy to be separated from each other. No adhesion exists between the patches and visceral organs, and after incision of the inside of the patches, new tissues are few. Microscopic results show that more collagen fibers are visible only on the surfaces of the patches, and there are no new tissues inside the patches, thus it can be seen, the growth of new tissues to the inside of the PVDF2 membrane is slower.

The surgical sites of the PP group are harder in touch, and the presence of foreign bodies can be obviously felt. There is a relatively thin layer of new tissue on the surface of each patch of the PP group. The new tissue and the skin are unclear in layers, and not easy to be separated from each other. The patches are close to viscera with serious adhesion to the viscera, and difficult to tear, and intestinal tract swells. Microscopic results show that a small quantity of fibroblasts and collagen are formed around the patches, with a relatively small amount of blood capillary hyperplasia ($\leq$2/HPF); and a large amount of infiltration of foreign body giant cells and lymphocytes shows obvious rejection and immune inflammatory response.

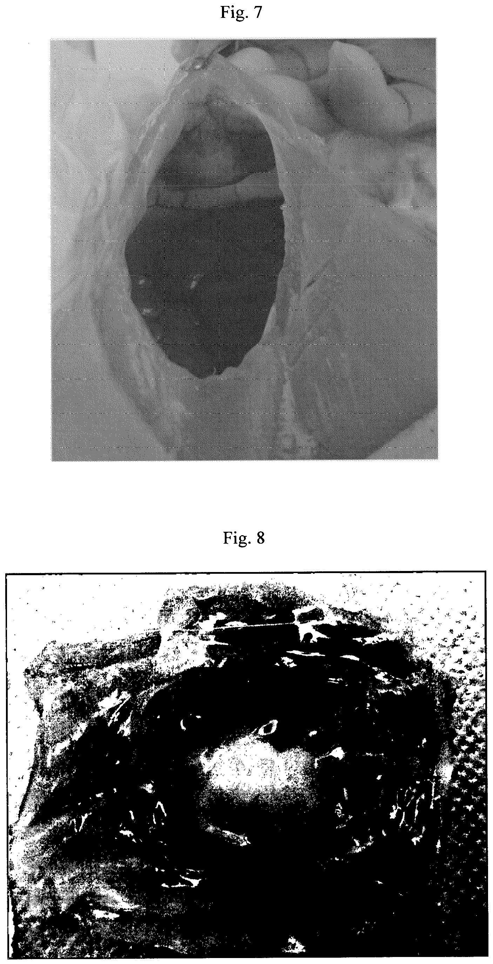

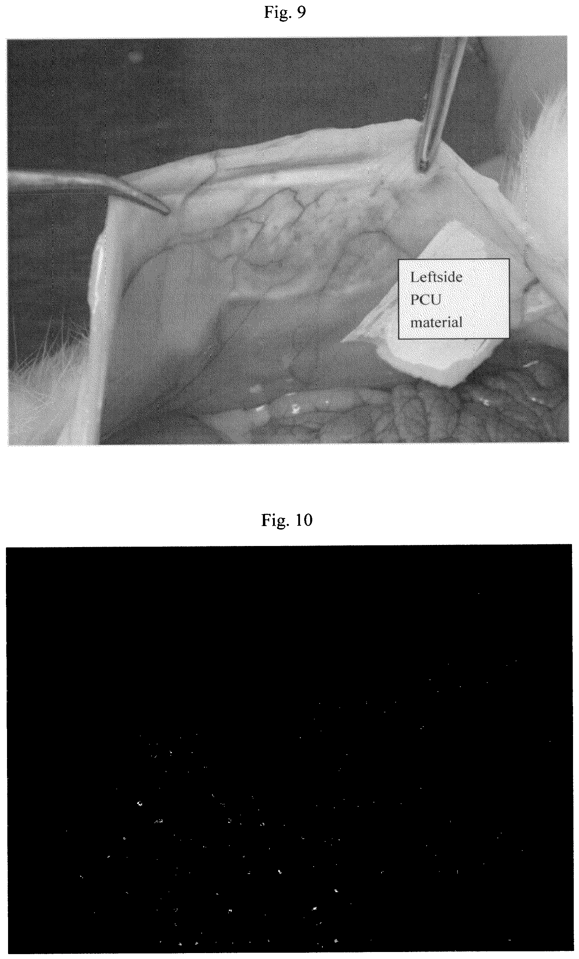

Six months after surgery, four animals in the PP group are dissected, and five animals in each of the other groups are dissected. The patches at repaired parts and tissues within the range of 2cm around the patches are incised. The surgical sites of the PVDF1 group and the PCU group are very soft in touch, as shown in Figure 6 and Figure 9, the patches has been integrated with new tissues which are difficult to be distinguished from the patches, the new tissues and the skin are clear in layers, and easy to be separated from each other. No adhesion exists between the new tissues and visceral organs. Microscopic observation results show that the peripheries of the parts implanted with the patches are tightly combined with collagen fibers and fibrocytes; and a large number of collagen fibers are visible inside the patches. The surgical sites of the PVDF2 group are softer in touch, as shown in Figure 7 and Figure 8, the patches and the new tissues still can be distinguished from each other. The new tissues and the skin are clear in layers, and easy to be separated from each other. No adhesion exists between the patches and visceral organs. Microscopic observation results show that more collagen fibers are visible on the surfaces of the patches, and after incision of the inside of the patches, there are still fewer new tissues.

The surgical sites of the PP group are harder in touch, and the presence of foreign bodies can be obviously felt. The patches of the PP group and the skin are unclear in layers, and cannot be separated from each other; serious adhesion exists between the patches and visceral organs; microscopic observation results show that a small quantity of fibroblasts and collagen are formed only around the patches, with a small amount of blood capillary hyperplasia ($\leq$5/HPF) and a large amount of infiltration of foreign body giant cells and lymphocytes visible on the surfaces, and foreign body reaction and immune rejection are serious; and this shows that the patches of the PP group are not conducive to cell adhesion and ingrowth, and have poor histocompatibility and repair effect.

Example 9 Application of fibrous membrane in pelvic floor repair

Miniature pig experiments are carried out by adopting a pelvic floor repair patch (PVDF3 patch) made from a fibrous membrane prepared in Example 3, a pelvic floor repair patch (PVDF4 patch) made from a fibrous membrane prepared in step 2 of Example 3, a pelvic floor repair patch (PCU1 patch) made from a fibrous membrane prepared in Example 6 and a pelvic floor repair patch made from a polypropylene material (PP patch, provided by the Guangzhou Overseas Chinese Hospital of Johnson&Johnson ProliftTM). All the patches are cut into 2cm$\times$2cm pieces. Eight female miniature pigs with weight of 20-25kg and sexual maturity are selected, and divided into four groups, namely PVDF3 group, PVDF4 group, PCU1 group and PP group, with two pigs in each group. The miniature pigs in the experiments are subjected to general anesthesia, and the abdominal walls of lower abdomens are incised to expose bladders, uteruses and upper sections of vaginas. The patches are respectively implanted between gaps of the vaginas and the bladders, and fixed by silk threads. The PVDF3 patch, the PVDF4 patch, the PCU1 patch and the PP patch are respectively used in the PVDF3 group, the PVDF4 group, the PCU1 group and the PP group serving as a control group. Postoperative routine feeding and observation for the animals are carried out. During the observation period, all the experimental animals are good in performance, and the incisions are healed well, without expulsion or exposure of implanted materials. The animals can take food and drink water normally after surgery, and all survive to the sampling time.

Four weeks after surgery, bladder and vagina interstitial tissue specimens, including the pelvic floor repair patches, are incised. For the PVDF3 group, the PVDF4 group, the PCU1 patch and the PP group, the surgical sites of the PP material is the hardest in touch, followed by the material of the PVDF4 group, the material of the PCU1 group is soft, and the material of the PVDF3 group is the softest with toughness. The surfaces of the patches of the PVDF3 group, the PVDF4 group and the PCU1 group are respectively covered with a layer of new epithelium, the patches are not easy to be torn from new tissues, new blood capillaries are visually, the surfaces of the patches in the PP group are also respectively covered with a layer of new tissues, and the patches are easy to be separated from the new tissues. Pathological results show that there is a large amount of hyperplasia of fibroblasts and collagen fibers inside the patches of the PVDF3 group and on the surfaces thereof, and more blood capillaries (5-10/HPF) exist inside the patches, thus it can be seen, new tissues have grown into the membrane at a relatively high speed. There are more fibroblasts and collagen fibers on the surfaces of the patches in the PCU1 group and the PVDF4 group, and a small amount of hyperplasia of fibroblasts and collagen fibers, as well as a small amount of blood capillary hyperplasia (3-5/HPF) are visible inside the patches, it shows that new tissues have grown into the membrane at a relatively low speed. A little of lymphocyte infiltration is visible inside the patches in the PVDF4 group, with a mild immune inflammatory response. There are more fibroblasts and collagen, a relatively small number of blood capillaries (2-3/HPF), and a small number of foreign body giant cells ($\leq$3/HPF) around the patches in the PP group, thus it can be seen, new tissues cannot grow into a whole with the membrane, and repair is slower, with foreign body rejection reaction.

Twelve weeks after surgery, vagina and bladder samples, including the pelvic floor repair implanted materials, are incised. For the PVDF3 group, the PVDF4 group, the PCU1 group and the PP group, the PP material is the hardest in touch, followed by the PVDF4 material, and there is a relatively obvious difference between softness of the repaired parts and surrounding tissues; and the PCU1 material is soft in touch, and the PVDF3 is the softest with toughness. A large quantity of fibroblasts and collagen are visible on the surfaces of the patches in the PVDF3 group and inside such patches, and the patches are integrated with grown tissues and cannot be distinguished and separated from them. Microscopic observation results show that the peripheries of the parts implanted with the patches are tightly combined with collagen fibers and fibrocytes, new tissues cannot be distinguished from the membrane material, a large number of collagen fibers are visible inside the patches, and the new tissues have grown into a whole with the membrane completely to realize regeneration and repair. The patches of the PCU1 group are not easy to be separated from the new tissues which have grown into the material visually, and the material is difficult to be distinguished from the new tissues. Microscopic observation results show that a large number of fibroblasts and a small number of blood capillaries (3-5/HPF) grow on the surfaces of the patches, and a relatively small number of blood capillaries (2-3/HPF) are visible inside the patches. The patches of the PVDF4 group can be separated from the new tissues, more fibroblasts and a small number of blood capillaries (3-5/HPF) grow on the surfaces of the patches, and a relatively small number of blood capillaries (≤2/HPF) are visible inside the patches. Growth of more fibroblasts and a relatively small number of blood capillaries (2-3/HPF) is also visible on the surfaces of the patches in PP group, but the patches can be easily separated from grown tissues, thus it can be seen, the new tissues cannot grow into a whole with the membrane, and the repair effect is poorer.

It can be seen, the fluffiness of the implanted patches plays an important role in quick adhesion of cells and tissues to the surfaces of the implanted patches and their growth into the materials, and high fluffiness and softness of the patches are more advantageous to the integration of the new tissues and the materials; and lower fluffiness will lead to relatively slow growth of the new tissues into the membrane.

Example 10 application of fibrous membrane in dura repair

A fibrous membrane used for tissue repair prepared in Example 4 and a fibrous membrane prepared in step (2) of Example 4 are cut into 4cm x 6cm pieces which are cleaned, sterilized and respectively made into a PLLA1 dura repair patch (made from the fibrous membrane of Example 4), and a PLLA2 dura repair patch (made from the fibrous membrane obtained in step (2) of Example 4), and a commercial clinically used dura repair patch of animal origin (NAOMOJIAN, a sample provided by the Third Affiliated Hospital of Sun Yat-Sen University) is adopted in a control group.

Six male or female healthy dogs are selected, with the weight being 10-15kg, and an observation period of 2-3 months. General anesthesia and double-top craniotomy are carried out for each dog in the experiment, with man-made bilateral partial dura defect and injury of brain tissue, and then dura neoplasty is implemented on both sides of the same experimental dog's brain respectively by using the PLLA1 dura repair patch and the PLLA2 dura repair patch, with three dogs in parallel experiments. The dura neoplasty is implemented on both sides of the same experimental dog's brain respectively by using the PLLA2 dura repair patch and the dura repair patch of animal origin in the control group, with three dogs in parallel experiments. Postoperative routine feeding and observation for the dogs are carried out, specimens are collected on the repair patches at the end of each observation period, and comparative observation on gross specimens and microscopic tissues is carried out. After being fed for a set period of time, all the experimental animals are anesthetized, skulls are exposed according to the aforementioned craniotomy, the external surfaces of the repair patches are separated and exposed, the animals are sacrificed through intravenous injection of air, and after the skulls are sawn, the repair patches and surrounding tissues are opened and incised. The external surfaces and texture of the repair patches, their relationships with surrounding tissues, the presence of cysts or induration, and adhesion between the internal surfaces and brain tissues are visually observed. The specimens are bottled and soaked in formalin stationary liquid, and the specimen bottle is labeled. After formalin fixation is carried out at room temperature for one week, local tissues at surgical sites are taken and embedded with conventional paraffin, and histological sections are stained with hematoxylin and eosin (HE).

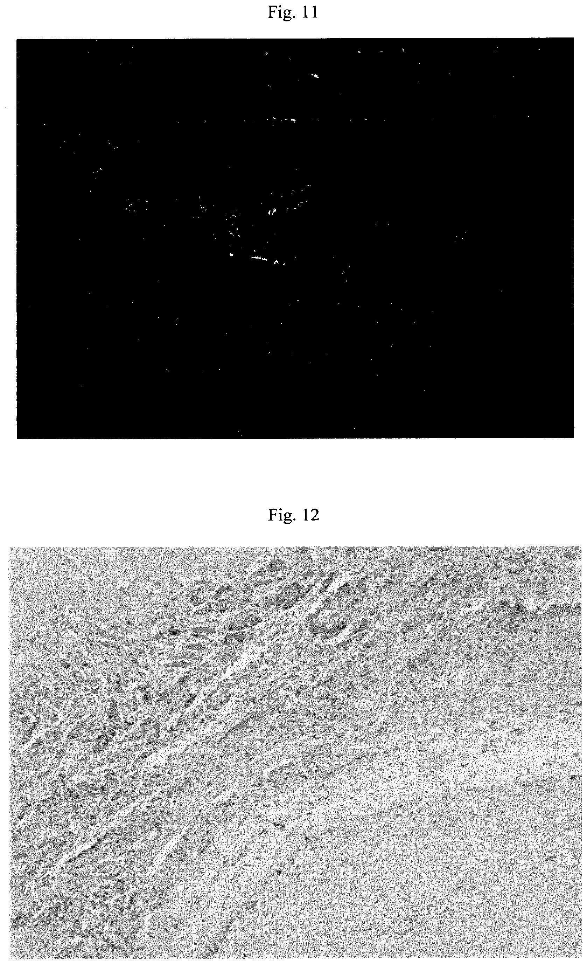

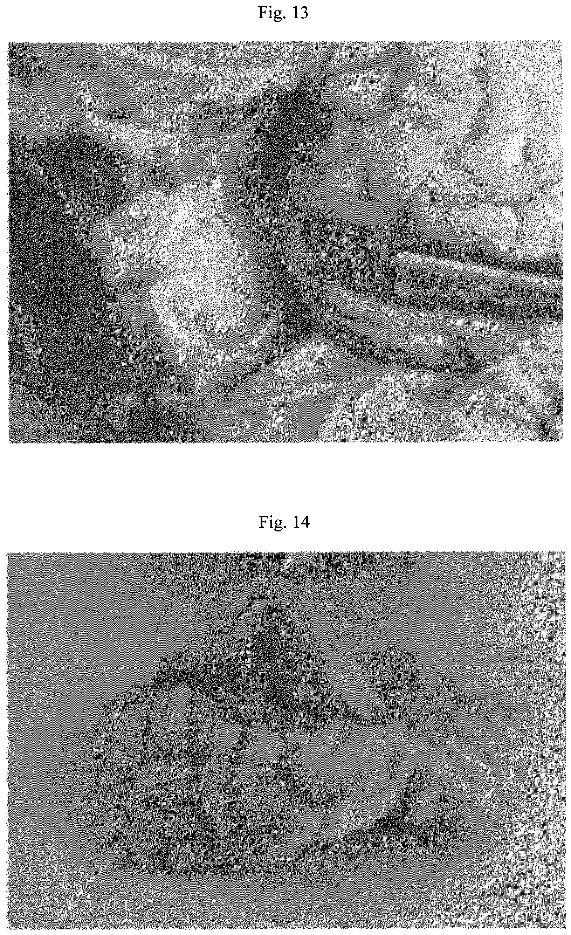

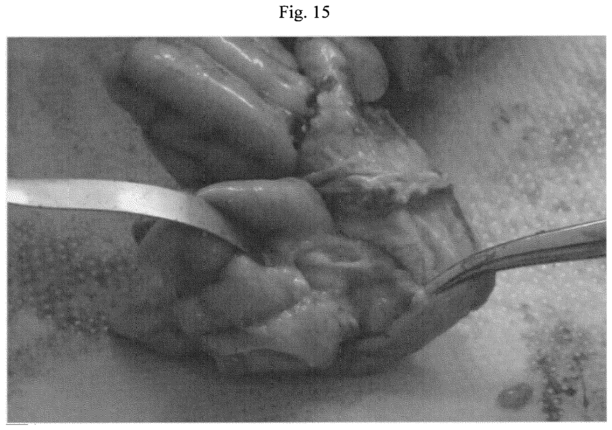

The six dogs are recovered well after surgery, and the incisions are healed well without secretion. After surgery, the dogs can take food and drink water normally, and their outdoor activities are normal, without dyskinesia. Three months later, the dogs are sacrificed through intravenous injection of air, and specimens are incised within the range of greater than 1cm around the surgical sites by taking the surgical sites as centers, so that the specimens include the repair patches, peripheral dura and brain tissues on the internal surfaces. After the specimens are incised out, skulls and meninges are separated from each other layer by layer, it can be seen, the meninges at the parts implanted with the PLLA1 dura repair patches are complete, the parts implanted with the patches have been completely replaced with fibrous tissues, the implanted materials are invisible, the meningeal internal surfaces at the implantation sites do not have adhesion, and brain tissues corresponding to the meninges have smooth surfaces without adhesion. The repair effect is shown in Figure 13 which is an anatomical figure, and a pathologic figure is shown as Figure 10; the meninges at the parts implanted with the PLLA2 dura repair patches are complete, it can be seen, partial implanted materials have been split into fragments to form new fibrous tissues, there is a little adhesion on the surfaces of brain tissues corresponding to the meninges, the repair effect is shown in Figure 14 which is an anatomical figure, and a pathologic figure is shown as Figure 11. While complete undegraded implanted patches are still visible at the parts implanted with the dura repair patches of animal origin in the control group, and there is more adhesion between the meningeal internal surfaces at the implantation sites and the brain tissues, which will cause complications such as ectopic discharge of brain tissues and seizures, the repair effect is shown in Figure 15 which is an anatomical figure, and a pathologic figure is shown as Figure 12.

Example 11 Application of fibrous membrane in skin repair

The fibrous membrane used for tissue repair prepared in Example 4 is cut into 5cm x 5cm pieces which are cleaned, sterilized and made into artificial skin for carrying out animal experiments. There are a total of 12 male or female experimental rabbits, weighing 2-2.5kg, and aged 6-8 months. The experimental rabbits are randomly divided into two groups (an experimental group and a control group), with six rabbits in each group. After intravenous anesthesia for ear margin of each rabbit, the animal is placed on a special operating table, and then subjected to skin preparation and disinfection. The full-thickness skin on the back is excised with a scalpel, with the excision area of 4cm x 4cm. Then artificial dermis is attached: for the experimental group, the artificial skin made from the fibrous membrane of Example 4 is pasted on a trauma, and fixed with suture lines at interval of 1cm. Blank control treatment is carried out for the control group. For both the experimental group and the control group, wounds are respectively covered with a layer of sterile oil gauze and sterile gauze which are fixed with the surrounding skin by using suture lines.

After surgery, animals are observed to find out whether their feeding, drinking and body temperature are normal or not, and the time of separation of covering from the wounds of each group, other physiological activities as well as the repair of the wounds are also observed.

At the two, four and eight weeks after surgery, two rabbits of each group are sacrificed, the entire wound on the back and adjacent normal skin are taken, fixed with formalin, and stained with HE, and neogenesis of tissues in dermis, inflammatory reaction, the thickness of epidermis structure and regeneration of appendant organs are observed under a light microscope.

All the wounds of the rabbits in the control group are adhered to the oil gauze or the gauze after surgery. Shedding of auxiliary materials exists, and the wounds bleed, with a lot of tissue fluid exudation. After being healed completely, the wounds have linear scabs. For the experimental group, the wounds are dry after surgery, without adhesion to the oil gauze or the gauze which is closely clung to the wounds. The wounds are not reddish, swollen or hot, without skin irritation or infection of the animals. There is no necrosis phenomenon under the covering materials. After being healed completely, the wounds have rectangular scabs. Through observation under a microscope, prickle cell layers of skin on healed wounds are thickened, basal cell hyperplasia is active, and cuticles of cuticular layers, acanthosis layers of granular layers and basal layers are all visible. The appendant organs of dermis layers, sweat glands, hair follicles and other structures are visible, it can be seen, a large number of fibroblasts are arranged parallel to the surface of the skin, among which there is more blood capillary hyperplasia, and a small amount of local lymphocytic infiltration.

Observation results show that the anti-infective capability and effect of preventing wound bleeding of the experimental group are superior to those of the control group. For the experimental group, the wounds are not reddish or swollen, without necrosis. Histological observation shows that the artificial skin material made from the fibrous membrane of Example 4 has better capability of promoting skin structure regeneration compared with the control group.

Example 12 Application of fibrous membrane in bone filling

The fibrous membrane used for tissue repair prepared in Example 2 is cleaned, sterilized and made into a bone repair stent for carrying out rabbit animal experiments. There are three New Zealand rabbits, weighing 2.5±0.5Kg, including one female rabbit, and two male rabbits. Skin preparation is carried out after general anesthesia, each animal is placed on a special operating table in a prone position, disinfected with iodophor alcohol, and sterile dressing is laid and fixed with a pair of towel forceps. Skin on the rabbit's leg is incised, periosteum is separated by using a detacher to expose tibia bone lamella, a 1cm x 2cm tibia defect of the rabbit is made by using a high-speed bone drill, a bone repair stent prepared from the fibrous membrane of Example 2 is folded to be fan-shaped and inserted into the defect, and the height of the filler is adjusted to enable the filler to be flush with the bone defect surface for suture. At 14 days after surgery, through visual observation, bone trabecula is relatively thick, through testing of an ultrasonic bone mineral density instrument, new sclerotin is compact, and more woven bones are formed. Three months after surgery, porosis occurs on the surface of a defective bone cavity. During percussion, poroma is hard in texture, with hardness similar to that of normal bone tissues, and its color is consistent with that of autologous bones. No inflammatory response occurs during recovery. After surgery, the animals are recovered well, and can take food and drink water normally. After the limb movement function is recovered gradually, dyskinesia is not found.

The above examples are sufficient to illustrate the beneficial effects brought about when the fluffiness of the fibrous membrane of the invention is 200-2,000cm$^3$/g, which can be summarized as follows:

Table 1

| Application types | Application for hernia repair | | | | Application for pelvic floor repair | | | | Application for dura repair | | |
|---|---|---|---|---|---|---|---|---|---|---|---|
| Materials | PCU | PVDF1 | PVDF2 | PP | PVDF3 | PCU1 | PVDF4 | PP | PLLA1 | PLLA2 | Animal origin |
| Fluffiness ($cm^3/g$) | 970 | 1,300 | 170 | 85 | 1,530 | 230 | 170 | 76 | 1,100 | 130 | 180 |
| Softness (mN) | 250 | 450 | 930 | 1,340 | 420 | 470 | 930 | 1,100 | 400 | 870 | 930 |
| Repair effects | A | B | C | D | E | F | G | H | I | J | K |

A: Very soft, with good compliance with tissues. New tissues rapidly grow inside and outside the patches, without adhesion to visceral tissues.

B: Very soft, with good compliance with tissues. New tissues rapidly grow inside and outside the patches, without adhesion to visceral tissues.

C: Relatively soft, with general compliance with tissues. New tissues slowly grow inside and outside the patches, without adhesion to visceral tissues.

D: Relatively hard, with poorer compliance with tissues. No new tissues grow inside and outside the patches, with serious adhesion to the tissues.

E: Very soft, with toughness. New tissues rapidly grow, and the patches are integrated with the new tissues, which cannot be distinguished or separated from each other.

F: Soft, new tissues grow relatively fast, and the patches and the new tissues are not easy to be separated from each other.

G: Relatively soft, new tissues slowly grow, and the patches and the new tissues can be distinguished and separated from each other, but relatively difficult.

H: Relatively hard, no new tissues grow inside and outside the patches, and the patches and the tissues are relatively easy to be separated from each other.

I: Soft, new tissues can be rapidly formed to repair dura, the material is degraded relatively fast, and no adhesion occurs between the new tissues and brain tissues.

J: Relatively hard, growth of new tissues and dura repair are relatively slow compared with the PLLA1 material, the material is degraded relatively slow, and a little adhesion occurs between the new tissues and brain tissues.

K: Relatively hard, growth of new tissues and dura repair are slow compared with the PLLA2 material, the material is degraded relatively slow, and more adhesion occurs between the new tissues and brain tissues.

It can be seen from the table above, the tissue repair patches with the fluffiness controlled to be 200-2,000cm$^3$/g can induce rapid growth of cells in the patches to form new tissues so as to achieve the repair effect. Also it can be seen that for the biodegradable material (PLLA), compared with the patches with the fluffiness beyond the range of 200-2,000cm$^3$/g, the patches with the fluffiness within this range have faster repair effect, and the material is degraded faster. Further, after being repaired by using the patches with the softness within the range of 50-500mN, the postoperative comfort is significantly improved due to relative softness of the repaired parts.

**<Preparation of implantable fibrous membrane used for repair: Examples 13-21>**

Example 13

(1) The PVDF is dissolved in a mixed solvent of N,N-dimethyl formamide/acetone with the volume ratio of 4:6, and the concentration of the PVDF in the solution is adjusted to be 18g/100mL to obtain a homogeneous fibrous material solution A; and the PLLA is dissolved in an HFIP solution, and the concentration of the PLLA in the solution is adjusted to be 5g/100mL to obtain a homogeneous fibrous material solution B.
(2) The above two kinds of homogeneous fibrous material solutions A and B are respectively put into five electrostatic spinning injection syringes, wherein four injection syringes contain the PVDF solution, and one injection syringe contains the PLLA solution. Four injection needles corresponding to the PVDF solution and one injection needle corresponding to the PLLA solution are uniformly arranged on a high-voltage power panel, the rate of a micro-injection pump is adjusted to be 5ml/h, the voltage of a high-voltage generator is adjusted to be 30KV, the receiving distance of a receiving device is adjusted to be 25cm, a fibrous membrane formed by two kinds of fibrous filament materials with different solubility properties intricately intersecting is prepared through simultaneous electrostatic cospinning of the two materials, and electrostatic spinning is turned off after the fibrous membrane is 0.5mm thick.
(3) The membrane which is taken down is put into an HFIP solvent for ultrasonic swelling and dissolving for 6h to enable the PLLA material therein to be dissolved completely to prepare a fluffy fibrous layer (A1).

The average diameter of fibers of the fluffy fibrous layer (A1) is 3$\mu$m, the membrane thickness is 0.5mm, the average pore size is 350$\mu$m, the tensile strength is 60N/cm, the fluffiness is 420cm$^3$/g, and the softness is 470mN.

Example 14

(1) The PVDF material is dissolved in a mixed solvent of N,N-dimethyl formamide/acetone with the volume ratio of 4:6, and the concentration of the PVDF in the solution is adjusted to be 20g/100mL to prepare a homogeneous fibrous material solution.
(2) The fibrous material solution obtained in step (1) is put into an electrostatic spinning injection syringe, the rate of a micro-injection pump is adjusted to be 6ml/h, the voltage of a high-voltage generator is adjusted to be 30KV, the receiving distance of a receiving device is adjusted to be 25cm, fibrous filaments are obtained by electrostatic spinning, and received into a membrane-like structure, and the electrostatic spinning is turned off after the membrane is 0.5mm thick to obtain a fibrous membrane.
The average diameter of fibers of the fibrous membrane obtained herein is 2$\mu$m, the membrane thickness is 0.5mm, the average pore size is 150$\mu$m, the tensile strength is 32N/cm, the fluffiness is 110cm$^3$/g, and the softness is 740mN.
(3) The fibrous membrane prepared in step (2) is treated according to a method identical with step (3) of Example 3 to obtain a fluffy fibrous layer (A2).

The average diameter of fibers of the fluffy fibrous layer (A2) is 2$\mu$m, the membrane thickness is 0.6mm, the average pore size is 380$\mu$m, the tensile strength is 37N/cm, the fluffiness is 1,105cm$^3$/g, and the softness is 400mN.

Example 15

(1) The PVDF material is dissolved in a mixed solvent of N,N-dimethyl formamide/acetone with the volume ratio of 4:6, and the concentration of the PVDF in the solution is adjusted to be 18g/100mL to prepare a homogeneous fibrous material solution.

(2) The fibrous material solution obtained in step (1) is put into an electrostatic spinning injection syringe, electrostatic spinning is carried out according to a method the same as step (2) of Example 14 to obtain a fibrous membrane, except for the receiving distance of a receiving device is adjusted to be 20cm.

The average diameter of fibers of the fibrous membrane obtained herein is $2\mu$m, the membrane thickness is 0.5mm, the average pore size is $190\mu$m, the tensile strength is 43N/cm, the fluffiness is 170cm$^3$/g, and the softness is 930mN.

(3) And then two sides of the fibrous membrane are clamped with a fixture, and stretched at the temperature of 95 DEG C according to a method the same as step (3) of Example 4 to obtain a fluffy fibrous layer (A3).

The average diameter of fibers of the fluffy fibrous layer (A3) is $2\mu$m, the membrane thickness is 0.6mm, the average pore size is $400\mu$m, the tensile strength is 65N/cm, the fluffiness is 1,410cm$^3$/g, and the softness is 400mN.

Example 16

(1) The PCU is dissolved in a mixed solvent of N,N-dimethyl formamide and tetrahydrofuran, the concentration of the PCU in the solution is adjusted to be 12g/100mL, and the mixing ratio (volume ratio) of the N,N-dimethyl formamide to the tetrahydrofuran is adjusted to be 1:1 to obtain a homogeneous fibrous material solution A.

The PLLA is dissolved in an HFIP solution, and the concentration of the PLLA in the solution is adjusted to be 5g/100mL to obtain a homogeneous fibrous material solution B.

(2) The above two kinds of homogeneous fibrous material solutions A and B are respectively put into four electrostatic spinning injection syringes, wherein three injection syringes contain the PCU solution, and one injection syringe contains the PLLA solution. Three injection needles corresponding to the PCU solution and one injection needle corresponding to the PLLA solution are uniformly arranged on a high-voltage power panel, the rate of a micro-injection pump is adjusted to be 6ml/h, the voltage of a high-voltage generator is adjusted to be 28KV, the receiving distance of a receiving device is adjusted to be 22cm, a fibrous membrane formed by two kinds of fibrous filament materials with different solubility properties intricately intersecting is prepared through simultaneous electrostatic cospinning of the two materials, and electrostatic spinning is turned off after the fibrous membrane is 0.5mm thick.

(3) The membrane which is taken down is put into an HFIP solvent for ultrasonic swelling and dissolving for 6h to enable the PLLA material to be dissolved completely, while the PCU material remains unchanged, and the insoluble material is taken out from the solvent to obtain a fluffy fibrous layer (A4).

The average diameter of fibers of the fluffy fibrous layer (A4) obtained herein is $2\mu$m, the membrane thickness is 0.5mm, the average pore size is $300\mu$m, the tensile strength is 25N/cm, the fluffiness is 830cm$^3$/g, and the softness is 230mN.

Example 17

A fluffy fibrous layer (A5) is obtained according to a method identical with Example 4.

The average diameter of fibers of the fluffy fibrous layer (A5) is $2\mu$m, the membrane thickness is 0.6mm, the average pore size is $450\mu$m, the tensile strength is 50N/cm, the fluffiness is 1,100cm$^3$/g, and the softness is 400mN.

Example 18

The PVDF material is dissolved in a mixed solvent of N,N-dimethyl formamide/acetone with the volume ratio of 4:6, and the concentration of the PVDF in the solution is adjusted to be 20g/100mL to prepare a homogeneous fibrous material solution.

The fluffy fibrous layer (A2) prepared in Example 14 covers the surface of a receiving roller, the above PVDF solution is put into an electrostatic spinning injection syringe, the rate of a micro-injection pump is adjusted to be 4ml/h, the voltage of a high-voltage generator is adjusted to be 28KV, the receiving distance of a receiving device is adjusted to be 20cm, the movement speed of an electrospinning needle is adjusted to be 10cm/s, and the rotating speed of the receiving roller is 4,000 revolutions/min. Electrostatic spinning is carried out to form a directional fibrous layer (B1) on the fluffy fibrous layer (A2) so as to obtain a double-layer fibrous membrane comprising the fluffy fibrous layer (A2) and the directional fibrous layer (B1).

Example 19

(1) The PVDF material is dissolved in a mixed solvent of N,N-dimethyl formamide/acetone with the volume ratio of 4:6, and the concentration of the PVDF in the solution is adjusted to be 20g/100mL to prepare a homogeneous fibrous material solution.

The above solution is put into an electrostatic spinning injection syringe, the rate of a micro-injection pump is adjusted to be 4ml/h, the voltage of a high-voltage generator is adjusted to be 28KV, the receiving distance of a receiving device is adjusted to be 20cm, the movement speed of an electrospinning needle is adjusted to be 10cm/s, the rotating speed of a receiving roller is 4,000 revolutions/min, and electrostatic spinning is carried out. The electrostatic spinning is turned off after the membrane thickness is about 0.3mm to obtain a single-layer directional fibrous layer (B2) with ordered orientation.

(2) The two kinds of fibrous membranes, namely the directional fibrous layer (B2) obtained in step (1) and the fluffy fibrous layer (A3) prepared in Example 15 are superposed together, and then all the above layers are connected together by means of point ultrasonic fusion at intervals of 10cm by using 20,000Hz frequency ultrasounds (Futan Mechanical Equipment Co., Ltd., type: JT-200-S) to obtain a double-layer fibrous membrane comprising the fluffy fibrous layer (A3) and the directional fibrous layer (B2).

Example 20

<Hemostatic layer contained in surface>

The 0.9g of sodium chloride and 1.79g of disodium hydrogen phosphate dodecahydrate are fully dissolved in 70mL of aqueous solution, and then 1.5mL of acetic acid solution with concentration of 36% (in volume) and 20mL of ethanol are added and stirred evenly to obtain a solution A.

The 2g of oxidized cellulose and 2g of type II collagen are dissolved in the above solution A by heating, and cooled to obtain a solution B.

Lyophilized thrombin powder is prepared to have the concentration of 350 units/mL, and 10mL of thrombin solution is added into the above solution B to obtain a solution C.

The fluffy fibrous layers (A1-5) prepared in Examples 13, 14, 15, 16 and 17 are immersed in the above solution C for 10min, and the sufficiently soaked membranes are freeze-dried overnight and cut into pelvic floor repair membranes containing hemostatic functional component oxidized cellulose and tension-free slings of required specifications.

**<Application of implantable membrane used for tissue repair in pelvic floor repair: Examples 21-22>**

Example 21

Miniature pig experiments are carried out by adopting an existing clinically used PP mesh (3DMAX™ Mesh, a sample provided by the Second Affiliated Hospital of Sun Yat-Sen University) as a control group (group I), a fibrous membrane obtained by cutting the electrospinning membrane prepared in step (2) of Example 13 as a control group (group II), as well as an implantable membrane 1 (group III) obtained by cutting the PVDF fluffy fibrous layer (A1) prepared in Example 13, an implantable membrane 2 (group IV) obtained by cutting the PVDF fluffy fibrous layer (A2) prepared in Example 14, an implantable membrane 3 (group V) obtained by cutting the PVDF fluffy fibrous layer (A3) prepared in Example 15, an implantable membrane 4 (group VI) obtained by cutting the PCU fluffy fibrous layer (A4) prepared in Example 16, and an implantable membrane 5 (group VII) obtained by cutting the PLLA fluffy fibrous layer (A5) prepared in Example 17 as experimental groups. The above materials are all cut into 2cm x 2cm membranes. A total of 28 healthy female miniature pigs with sexual maturity, weighing 20kg-25kg, are selected and randomly divided into seven groups, with four pigs in each group.

Each miniature pig is subjected to general anesthesia and fixed in an overhead position. The abdominal wall of lower abdomen is incised to expose bladder, uterus and an upper section of vagina. The membranes are respectively implanted between gaps of the vagina and the bladder, and fixed with silk threads. Postoperative routine feeding and observation for the animals are carried out. During the observation period, all the experimental animals are good in performance, and the incisions are healed well, without protrusion or exposure of implanted materials, or reddening and swelling of surgical sites. The animals are normal in drinking and feeding, as well as mental state after surgery, and all survive to the sampling time in a space enough for activities.

Four weeks after surgery, two animals of each group are sacrificed, and vagina and bladder interstitial tissue specimens, including the repair patches, are incised. For the softness of implanted materials, the PP mesh in the control group I obviously becomes hard in touch, with foreign body sensation, followed by the softness of the control group II, and the softness of all the patches in the experimental groups is greatly improved. Specifically, the softness of the patches in groups IV, V and VII is optimal with toughness, and close to that of autologous tissues.

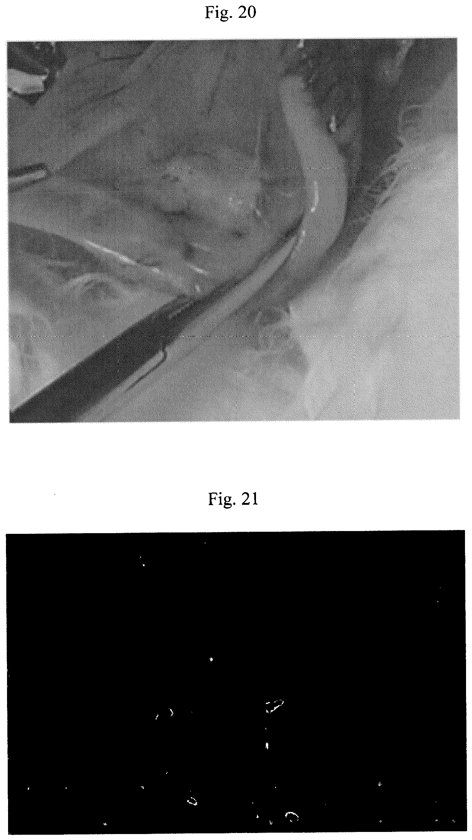

Through comparison between tissue ingrowth inside materials and growth with surrounding new tissues, by general observation, the tissue ingrowth and bonding strength with the surrounding new tissues of the membranes in the experimental groups (III-VII) are more excellent compared with the PP mesh in the control group I and the membrane in the control group II, which is manifested as follows: the surfaces of the membranes in the experimental groups are respectively covered with a layer of new epithelial tissues, the vascularization degree is higher, and the membranes and the new tissues are not easy to be separated from each other; wherein the anatomical effect after implantation of the PVDF material in group IV for four weeks is shown in Figure 20, it can be seen, there is no difference between the new tissues at implantation sites and surrounding tissues. The surfaces of the PP mesh in the control group I and the membrane in the control group II are also covered with a layer of new tissues, but blood capillaries on the new tissues are sparse; moreover, the PP mesh in the control group I is easy to be separated from the patch, as shown in Figure 21, after being separated from the new tissues, the material is still a grid that is harder in texture, without ingrowth of new tissues; it can be seen, the experimental groups have better cell and tissue ingrowth effect, as well as stronger compatibility and binding capability with the new tissues.

Pathological results show that there are a large amount of hyperplasia of fibroblasts and collagen fibers on the surfaces of the implantable membranes in groups III-VII and inside these membranes, and more blood capillaries (3-5/HPF) exist inside the patches; wherein a large number of blood capillaries (5-10/HPF) exist inside the patches in group V. Thus it can be seen, new tissues have grown into the membranes at a relatively high speed, the vascularization degree is high, and higher fluffiness is more advantageous to cell crawling and proliferation, as well as rapid tissue ingrowth. There are more fibroblasts and collagen fibers on the surfaces of the fibrous membranes in group II, and a small amount of hyperplasia of fibroblasts and collagen fibers, a little infiltration of lymphocytes (<5/HPF), as well as a small amount of blood capillary hyperplasia (2-3/HPF) are visible inside the patches, it shows that new tissues have grown into the membranes at relatively low speed, with a mild immune inflammatory response. There are a large number of fibroblasts and collagen, a relatively small number of blood capillaries (<2/HPF), and a small number of foreign body giant cells (≤3/HPF) around the PP mesh in group I, thus it can be seen, new tissues cannot grow into a whole with the membranes, and repair is slower, with foreign body rejection reaction.

Observation is continued till 12 weeks after surgery, during the period, the miniature pigs implanted with the PP mesh in group I show dysphoria, such as friction on inner walls and rails, and tail biting, and are in a poor mental state. The rest of the experimental miniature pigs are normal in activities, and are bred in a broad breeding site.

Twelve weeks after surgery, two animals of each group are sacrificed, and vagina and bladder specimens, including the pelvic floor repair implanted materials, are incised. Upon touching with hands, the PP mesh material in group I still remains certain hardness compared with that in four weeks after surgery, with obvious foreign body sensation; the fibrous membrane in group II is softer, but there is still obvious distinction between implantation sites and surrounding tissues in touch; the softness of the implantable membranes in experimental groups III-VII is basically close to that of autologous tissues, and there is basically no obvious difference between the parts implanted with the materials and surrounding tissues in handfeel; wherein the material in group V is almost consistent with the autologous tissues in touch.

Through visual observation, the materials of the implantable membranes in groups III-VII are integrated with grown tissues, cannot be distinguished or separated from the tissues, and are close to human tissues on the whole, and blood vessels can be clearly seen. Microscopic observation results show that the peripheries of the implantable membranes in groups III-VII are tightly combined with collagen fibers and fibrocytes, new tissues and membrane materials cannot be distinguished from each other, a large number of collagen fibers are visible inside the membranes, it can be seen, the new tissues have grown into a whole with the membranes completely, and reconstruction and repair are realized; and pathological results show that a large number of fibroblasts and collagen are visible on the surfaces of the materials and inside these materials, no foreign body giant cells or lymphocytes are visible, and the materials and new tissues are integrated, thus being difficult to distinguish. It can be seen, higher fluffiness is more advantageous to induction of cell ingrowth and tissue regeneration, suitable softness is more advantageous to binding of new tissues at implantation sites and surrounding tissues, and notably, the new tissues do not cause foreign body sensation in the body, or friction damage to new or autologous tissues.

Through visual observation, the fibrous membrane in group II can be separated from new tissues, and more blood capillaries are visible on the surface; microscopic observation results show that there is an obvious boundary between the periphery of the fibrous membrane in group II and collagen fibers and fibrocytes in combination, the new tissues and the membrane material are easy to be distinguished from each other, there are a large number of collagen fibers and rich blood capillaries on the surface, and the material is still visible inside the membrane; pathological results show that more fibroblasts and a large number of blood capillaries (5-10/HPF) grow on the surface of the material, a small number of blood capillaries (≤3/HPF) and foreign body giant cells (1/HPF) are visible inside the membrane, it can be seen, the new tissues have grown into the membrane, but the new tissues are relatively few, with mild foreign body response. For the PP mesh in group I, four-level adhesion (by adopting a Nari adhesion scoring method 1) occurs between the repaired parts and surrounding tissues, the material is easier to be separated from grown tissues, the PP material can be clearly seen, and no new tissue ingrowth or penetration is visible inside the material; and pathological results show that a small number of fibroblasts and collagen are formed only around the material, more blood capillary hyperplasia (3-5/HPF) and a large amount of infiltration of foreign body giant cells and lymphocytes are visible on the surface, and foreign body response and immune rejection are serious, it can be seen, the new tissues cannot grow into a whole with the membrane, and the repair effect is poor. Meanwhile, presumably the dysphoria of corresponding experimental pigs in the observation process may be caused by strong foreign body sensation and tissue infection response.

Example 22

A double-layer fibrous membrane (hereinafter referred to as disordered fluffy membrane) is prepared from a non-oriented fibrous layer (C) obtained by conventional electrospinning and a fluffy fibrous layer (A3) obtained in Example 15 by a method of step (2) in Example 19.

With the above disordered fluffy membrane as a control group, and the double-layer fibrous membrane (hereinafter referred to as ordered fluffy membrane) in Example 19 as an experimental group, animal experiments are carried out to verify the effect of an ordered structure.

A total of 12 healthy male or female New Zealand rabbits, weighing 2.0-2.5Kg, and aged about 6-12 months, are selected as experimental animals. The experimental rabbits are randomly divided into three groups, with four rabbits in each group. The membranes in the experimental group and the control group are all cut into 1cm x 4cm strips which are respectively implanted into abdomens of the rabbits subcutaneously, and tissue growth on the material surface is observed at two weeks and three months after surgery.

An implantation method comprises the following steps: skin preparation and disinfection are carried out for each rabbit's abdomen, abdominal skin is incised along a midline, subcutaneous fascia and muscles are bluntly separated to expose an appropriate material implantation range. Three 1cm x 4cm samples of the experimental group are implanted into the left side of an interlayer between subcutaneous fascia, and three 1cm x 4cm samples of the control group are implanted into its right side. The material is fixed at a corresponding position by using lines No.4, and a subcutaneous fascia layer and a skin layer are sutured respectively.

Two weeks later, fluffy surfaces of the experimental group and the control group are all wrapped with thin fibers, and implantation sites are rich in blood capillaries. A part of the material is taken out and dyed for observation, obvious cell growth and crawling orientation exist on the directional layer in the experimental group, while scattered cell growth exists on the non-directional layer in the control group. Three months later, a part of the two groups of samples are taken out for observation, obvious fibrous tissue directional texture exists on the directional fibrous layer in the experimental group, while no such directional texture exists on the non-directional fibrous layer in the control group. Thus it can be seen, the directional orientated fibrous layer (B) is more advantageous to guidance of cell crawling and tissue compliance growth, and repaired tissues are closer to natural fascia tissues.

**<Preparation of anti-adhesion tissue repair membrane: Examples 23-26>**

The fibrous membranes used for tissue repair prepared in Examples 1-3 are taken as fluffy fibrous layers (A'1-3) of anti-adhesion tissue repair membranes.

Example 23

(1) A PP woven mesh is selected, soaked in a PVDF/N,N-dimethyl formamide and acetone solution with concentration of 8g/100ml for 10min, taken out and dried in an air dry oven at 60 DEG C for 2h to obtain a woven mesh layer (C') wrapped with thin PVDF.
(2) The woven mesh layer (C') obtained in step (1) is put between two fibrous membranes used for tissue repair (fluffy fibrous layers (A'3)) obtained in Example 3, and placed on an ultrasonic welding machine together with the fibrous membranes, and ultrasonic welding is carried out for 8s under the conditions that the welding pressure is 120N and the frequency is 40KHz to obtain a tissue repair membrane base material.

Example 24

(1) A PP woven mesh is selected, soaked in a PCU/N,N-dimethyl formamide and tetrahydrofuran solution with concentration of 3g/100ml for 10min, taken out and dried in an air dry oven at 50 DEG C for 2h to obtain a woven mesh layer (C') wrapped with thin PCU.

(2) The woven mesh layer (C') obtained in step (1) is put between two fibrous membranes used for tissue repair (fluffy fibrous layers (A'1)) obtained in Example 1, and placed on an ultrasonic welding machine together with the fibrous membranes, and ultrasonic welding is carried out for 7s under the conditions that the welding pressure is 120N and the frequency is 40KHz to obtain a tissue repair membrane base material.

Example 25

(1) Chitosan with molecular weight of 600,000 is dissolved in an acetic acid aqueous solution with concentration of 5% (namely mass fraction of acetic acid), the concentration of the chitosan is 3% (w/v), and a light yellow solution is obtained.

(2) The above light yellow solution is placed into a spraying device, and the spraying device is sealed, externally connected with a pneumatic device, and assembled with a KZ-1 type hollow cone nozzle, the liquid outlet volume is adjusted to be 0.02ml/min, the spraying speed is adjusted to be 10cm/s, the spraying spacing is adjusted to be 15mm, and after the completion of setting, it is ready for spraying.

(3) By taking the fibrous membranes used for tissue repair (fluffy fibrous layers (A'1-3)) obtained in Examples 1-3 and samples obtained in Examples 23-24 as base materials, spraying is carried out on any one of the outer side surfaces of the fluffy fibrous layers. The spraying device is started to spray based on the parameters set in step (2), and after the spraying is carried out for 3-5 times, the samples are placed into an air dry oven at 35 DEG C for drying for 24h.

(4) The samples are taken out from the air dry oven, infiltrated with 75% alcohol, and then put into water to be soaked for 24h.

(5) The anti-adhesion tissue repair membranes excluding or containing intermediate layers are obtained by drying, cutting, packaging and sterilizing.

Example 26

(1) Carboxymethyl chitosan with molecular weight of 700,000 is dissolved in an acetic acid aqueous solution with concentration of 3% (namely mass fraction of acetic acid), the concentration of the carboxymethyl chitosan is 5% (w/v), and a solution with certain viscosity is obtained.

(2) The above solution is placed into a spraying device, and the spraying device is sealed, externally connected with a pneumatic device, and assembled with a general CC type fan-shaped nozzle, the liquid outlet volume is adjusted to be 0.01ml/min, the spraying speed is adjusted to be 15cm/s, the spraying spacing is adjusted to be 12mm, and after the completion of setting, it is ready for spraying.

(3) By taking the fibrous membranes used for tissue repair (fluffy fibrous layers (A'1-3)) obtained in Examples 1-3 and samples obtained in Examples 23-24 as base materials, spraying is carried out on any one of the outer side surfaces of the fluffy fibrous layers. The spraying device is started to spray based on the parameters set in step (2), and after the spraying is carried out for 5-7 times, the samples are placed into an air dry oven at 40 DEG C for drying for 24h.

(4) The samples are taken out from the air dry oven, infiltrated with 95% alcohol, and then put into water to be soaked for 24h.

(5) The anti-adhesion tissue repair membranes excluding or containing intermediate layers are obtained by drying, cutting, packaging and sterilizing.

**<Animal experiments of anti-adhesion tissue repair membrane>**

Example 27

Four groups of materials are selected from the anti-adhesion tissue repair membrane prepared in Example 25, and the materials in each group are respectively cut into 3.5cm x 6cm membranes for carrying out animal experiments for New Zealand rabbit's abdominal wall full-thickness defect repair.

The four groups of materials are respectively as follows:

Material No.1: an anti-adhesion tissue repair membrane prepared by combining Example 1 with Example 25;
Material No.2: an anti-adhesion tissue repair membrane prepared by combining Example 3 with Example 25;
Material No.3: an anti-adhesion tissue repair membrane prepared by combining Example 23 with Example 25;
Material No.4: an anti-adhesion tissue repair membrane prepared by combining Example 24 with Example 25;

There are a total of 20 New Zealand rabbits, weighing 2.5-2.8Kg, and aged 6-12 months. The experimental rabbits are randomly divided into four groups, namely a material group No.1, a material group No.2, a material group No.3 and a material group No.4 respectively, with five experimental animals in each group. Each animal in the experiment is anesthetized and subjected to skin preparation, lies on its back and is fixed on a board; after a surgical drape is disinfected, the skin at the center of the abdomen is incised by using a scalpel No.4 along a white line on the rabbit's abdomen to form an incision that is about 8cm long and expose a muscle layer of the abdominal wall, and the full-thickness abdominal wall with the size of 2cm x 5cm (including peritoneum, muscles and fascia tissues) is resected; and the material No.1, the material No.2, the material No.3 and the material No.4 are respectively sutured with muscles around the incisions of the abdominal walls of the corresponding New Zealand rabbits in each group by using silk threads 4/0 interruptedly. The silk threads 4/0 are also used for interrupted suture of the skin.

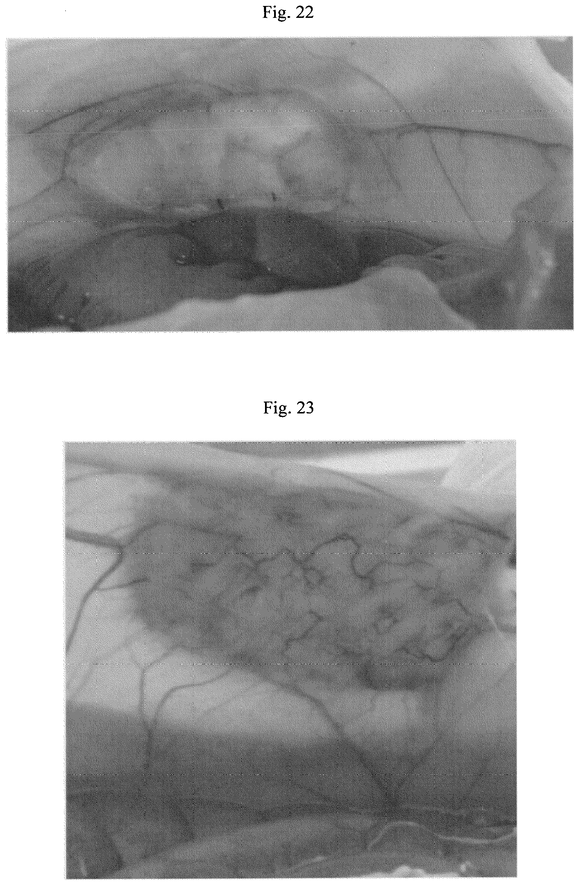

All the experimental animals are subjected to intramuscular injection of penicillin for one million IU/day within three days after surgery, and supplied with feed and water normally in cages. During the observation period, all the animals in the four groups can take food and drink water normally, the incisions are healed well, without infection of surgical sites or formation of abdominal bulge, and all the experimental animals can defecate normally, without intestinal obstruction, etc. No experimental animals die during the observation period. One month later, anatomy is carried out. Figure 22 shows an animal anatomical figure of the anti-adhesion tissue repair membrane (material No.1) prepared by combining Example 1 with Example 25; and Figure 23 shows an animal anatomical figure of the anti-adhesion tissue repair membrane (material No.3) prepared by combining Example 23 with Example 25. Observation results are shown in Table 2:

Table 2 Anatomical observation on four groups of materials

| Material groups | Anatomical description | Tensile strength |
|---|---|---|
| Material group No.1 | Soft in touch, with good compliance with tissues. New tissues grow around the patches, without adhesion to visceral tissues. | 27N/cm |
| Material group No.2 | Softer in touch, with good compliance with tissues. New tissues rapidly grow inside and outside the patches, without adhesion to visceral tissues. | 51 N/cm |
| Material group No.3 | Slightly hard in touch, tissues rapidly grow inside and outside the patches, without adhesion to visceral tissues. | 110N/cm |
| Material group No.4 | Slightly hard in touch, tissues rapidly grow inside and outside the patches, without adhesion to visceral tissues. | 108N/cm |

Experimental results show that all the above anti-adhesion tissue repair membranes can effectively promote tissue growth, the anti-adhesion effect of the membranes can be effectively realized by adopting a way of coating chitosan on surface layers, and the strength of the membranes can be greatly improved by means of ultrasonic welding of the PP material.

**Claims**

1. A fibrous membrane for use in tissue repair, wherein the fibrous membrane is formed by interweaving fibrous filaments with diameters of 10nm-100μm, of porous structure with the fluffiness of 200-2,000cm$^3$/g, whereby the average pore size of the fibrous membrane is 50-500μm, the thickness of the fibrous membrane is 0.2-2mm, its tensile strength is 10-300N/cm, and the softness of the fibrous membrane is 50-500mN.

2. The fibrous membrane according to claim 1, wherein the fibrous filaments can be made from biodegradable materials, non-biodegradable materials or their combination, wherein the biodegradable materials are synthetic biodegradable materials or natural polymeric materials; and the non-biodegradable materials are fluoropolymer materials, polypropylene materials or polyurethane materials.

3. The fibrous membrane according to claim 2, wherein the materials of the fibrous filaments are one or more of polylactic acid, polycaprolactone, polyglycolic acid, polylactic-co-glycolic acid, 1,3-propanediol polymer, collagen, gelatin, fibrin, silk-fibroin, elastin mimetic peptide polymer, chitosan, modified chitosan, polyvinylidene fluoride, polytetrafluoroethylene, polyurethane, polycarbonate polyurethane, polyether-based polyurethane, silane-modified polyurethane, polyethylene glycol, polyethylene terephthalate, polymethyl methacrylate, poly(3-hydroxybutyrate-co-3-hydroxyvalerate), poly(3-hydroxybutyrate-co-3-hydroxyhexanoate), polyphosphate, polyamino formic anhydride, polyesteramide, polyvinyl alcohol, poly(para-dioxanone), polycarbonate, starch, cellulose, alginate, chondroitin sulfate, heparin, glucosan and alginic acid.

4. A composite fibrous membrane according to any one of claims 1-3, wherein the fibrous membrane comprises a woven layer.

5. The composite fibrous membrane for use in tissue repair according to claim 4, wherein the woven layer is made from polypropylene, polyvinylidene fluoride, polytetrafluoroethylene, polyester, polylactic acid and polylactic-co-glycolic acid.

6. Method of preparing the fibrous membrane according to any one of claims 1-3, comprising the following steps:

   (1) dissolving two fibrous materials of different solubility properties in corresponding solvents to obtain two kinds of homogeneous fibrous material solutions;
   (2) placing the two kinds of homogeneous fibrous material solutions obtained in step (1) into different electrostatic spinning injection syringes to obtain a fibrous membrane formed by two kinds of fibrous filaments of different solubility properties mutually and intricately intersecting through electrostatic spinning;
   (3) According to the solubility properties of the materials of the fibrous filaments, dissolving one kind of fibrous filaments in the fibrous membrane produced in step (2) with a solvent, and keeping the other kind of fibrous filaments unchanged to obtain the fibrous membrane used for tissue repair.

7. Method of preparing the fibrous membrane according to any one of claims 1-3, comprising the following steps:

   (1) dissolving a fibrous material in a solvent to obtain a homogeneous fibrous material solution;
   (2) placing the homogeneous fibrous material solution obtained in step (1) into an electrostatic spinning injection syringe, carrying out electrostatic spinning to obtain fibers, and receiving the fibers into a membrane-like structure to obtain a fibrous membrane;
   (3) pre-freezing the fibrous membrane prepared in step (2) after being subjected to ultrasonic swelling with a solvent, and then carrying out vacuum freeze drying to obtain the fibrous membrane used for tissue repair.

8. Method of preparing the fibrous membrane according to any one of claims 1-3, comprising the following steps:

   (1) dissolving a fibrous material in a solvent to obtain a homogeneous fibrous material solution;
   (2) placing the homogeneous fibrous material solution obtained in step (1) into an electrostatic spinning injection syringe to obtain a fibrous membrane through electrostatic spinning;
   (3) stretching the fibrous membrane prepared in step (2) along the direction of horizontal axis or longitudinal axis of the fibrous membrane, and after stopping stretching, sizing the fibrous membrane in this stretching state; and then stretching the fibrous membrane along the direction perpendicular to the above stretching direction, and after stopping stretching, sizing the fibrous membrane in this stretching state to obtain a fluffy fibrous layer (A).

9. Method according to any one of claims 6-8, wherein during the electrostatic spinning in step (2), the rate of a microinjection pump is adjusted to be 0.1-15.0ml/h, the voltage of a high-voltage generator is adjusted to be 5-45KV, and the receiving distance of a receiving device is adjusted to be 5.0-30.0cm.

10. An implantable membrane for use in tissue repair in the treatment of female pelvic floor dysfunction comprising a fluffy fibrous layer (A), with a fluffiness of 400-1500cm$^3$/g, and a softness of 50-500mN, wherein the fluffy fibrous layer (A) is the fibrous membrane according to any one of claims 1-3.

11. The implantable membrane for use according to claim 10, wherein the implantable membrane further comprises a directional fibrous layer (B) which is a layer with a porous three-dimensional structure formed by the directional arrangement of fibrous filaments and wherein all layers in the implantable membrane are combined by means of electrostatic spinning, or ultrasonic fusion or suture.

12. The implantable membrane for use according to claim 11, wherein the diameters of the fibrous filaments in the directional fibrous layer (B) are 10nm-20$\mu$m.

13. The implantable membrane for use according to any one of claims 10-12, wherein the implantable membrane contains hemostatic drugs, anti-infective drugs, a cell growth regulator, a toxicity inhibitor, anesthetic micro-nano particles and/or drugs for the treatment of urinary incontinence.

14. The implantable membrane for use according to any one of claims 10-13, wherein the surface of the implantable membrane contains a hemostatic layer, wherein the hemostatic layer contains collagen, polyoxyethylene fiber, chitosan, fibrin, peptide and/or thrombin.

15. The implantable membrane for use according to claim 13 or claim 14, wherein the hemostatic layer is formed by methods of electrostatic spinning, freeze drying, air drying or vacuum drying.

16. The implantable membrane for use according to any one of claims 10-15, wherein the implantable membrane has through holes penetrating through the upper and lower surfaces of the implantable membrane.

17. Method for preparing the implantable membrane for use according to any one of claims 10-16 comprising preparing the fluffy fibrous layer (A) according to the method of any one of claims 6-9, wherein the method further comprises a step for preparing the directional fibrous layer (B) by electrostatic spinning.

18. An implantable medical device for use in the treatment of female pelvic floor dysfunction, comprising the implantable membrane according to any one of claims 10-16.

19. The implantable medical device for use according to claim 18, wherein the implantable medical device is a tension-free urinary incontinence sling or a pelvic floor patch.

20. The implantable medical device for use according to claim 19, wherein the tension-free urinary incontinence sling comprises a main body part and an end part, the main body part is made from the implantable membrane according to any one of claims 10-16, and the end part is used for surgical instrument traction and/or fixation.

21. The implantable medical device for use according to claim 20, wherein the main body part has a linear or corrugated outer contour.

22. The implantable medical device according to claim 21, wherein the pelvic floor patch comprises a central massive part according to any one of claims 10-16 for use in the treatment of pelvic organ prolapse and bulging, and the central massive part is made from the implantable membrane according to any one of claims 10-16, further comprising an arm-like structure which is positioned at the periphery of the central massive part for suspending a procident pelvic organ.

23. An anti-adhesion tissue repair membrane, wherein the surface layer on one side of the anti-adhesion tissue repair membrane is a fluffy fibrous layer (A'), and the surface layer on the other side is an anti-adhesion layer (B'); and wherein the fluffy fibrous layer (A') is the fibrous membrane according to any one of claims 1-3 and the anti-adhesion layer (B') contains chitosan and/or carboxymethyl chitosan.

24. The anti-adhesion tissue repair membrane according to claim 23, comprising an intermediate layer between the fluffy fibrous layer (A') and the anti-adhesion layer (B'), wherein said intermediate layer comprises a woven layer (C'), or a fibrous layer (D') or a combination thereof.

25. The anti-adhesion tissue repair membrane according to claim 24, wherein the fibrous layer (D') is formed by interweaving fibrous filaments with diameters of 10nm-100$\mu$m, and has a porous structure, with the fluffiness of 200-2,000cm$^3$/g.

26. The anti-adhesion tissue repair membrane according to claim 24 or claim 25, wherein the anti-adhesion layer (B') is combined with other layers by means of spray coating or ultrasonic welding.

27. Method for preparing the anti-adhesion tissue repair membrane according to any one of claims 23-26, comprising: preparing the fluffy fibrous layer (A') according to any one of claims 6-9, and combining the anti-adhesion layer (B') with other layers by means of spray coating or ultrasonic welding.

28. Use of the fibrous membrane according to any one of claims 1-3, or of the composite fibrous membrane according to claim 4 or 5, or of the anti-adhesion tissue repair membrane according to any one of claims 24-26 in the preparation of a hernia repair patch, a treatment system of female pelvic floor dysfunction, an artificial rotator cuff, a dura repair patch, a spinal meninges repair patch, artificial skin, a pericardium patch, artificial blood vessels, artificial nerve conduits, artificial periodontium, artificial ligament, artificial achilles tendon and bone repair products, wherein the treatment system for female pelvic floor dysfunction is a pelvic floor repair patch, a urinary incontinence sling or a fistula repair patch.

29. Hernia repair patch, pelvic floor repair patch, a urinary incontinence sling, a fistula repair patch, an artificial rotator cuff, a dura repair patch, a spinal meninges repair patch, artificial skin, a pericardium patch, artificial blood vessels, artificial nerve conduits, artificial periodontium, artificial ligament, artificial achilles tendon and bone repair products according to any one of claims 1-3, 8, 9, or 24-27.

**Patentansprüche**

1. Fasermembran zur Verwendung bei einer Gewebereparatur, wobei die Fasermembran durch Verweben von Faserfilamenten mit Durchmessern von 10 nm bis 100 $\mu$m gebildet wird und eine poröse Struktur mit einer Flaumigkeit von 200 bis 2000 cm$^3$/g aufweist, wobei die durchschnittliche Porengröße der Fasermembran 50 bis 500 $\mu$m beträgt, die Dicke der Fasermembran 0,2 bis 2 mm beträgt, deren Zugfestigkeit 10 bis 300 N/cm beträgt und die Weichheit der Fasermembran 50 bis 500 mN beträgt.

2. Fasermembran nach Anspruch 1, bei der die Faserfilamente aus biologisch abbaubaren Materialien, nicht biologisch abbaubaren Materialien oder deren Kombination hergestellt sein können, wobei die biologisch abbaubaren Materialien synthetische biologisch abbaubare Materialien oder natürliche polymere Materialien sind; und die nicht biologisch abbaubaren Materialien Fluorpolymermaterialien, Polypropylenmaterialien oder Polyurethanmaterialien sind.

3. Fasermembran nach Anspruch 2, bei der die Materialien der Faserfilamente eines oder mehrere von Polymilchsäure, Polycaprolacton, Polyglykolsäure, Polymilch-co-glykolsäure, 1,3-Propandiolpolymer, Kollagen, Gelatine, Fibrin, Seidenfibroin, Elastin-mimetisches Peptidpolymer, Chitosan, modifiziertes Chitosan, Polyvinylidenfluorid, Polytetrafluorethylen, Polyurethan, Polycarbonat-Polyurethan, Polyurethan auf Polyetherbasis, Silan-modifiziertes Polyurethan, Polyethylenglykol, Polyethylenterephthalat, Polymethylmethacrylat, Poly(3-hydroxybutyrat-co-3-hydroxyvalerat), Poly(3-hydroxybutyrat-co-3-hydroxyhexanoat), Polyphosphat, Polyaminoameisensäureanhydrid, Polyesteramid, Polyvinylalkohol, Poly(para-dioxanon), Polycarbonat, Stärke, Zellulose, Alginat, Chondroitinsulfat, Heparin, Glukosan und Alginsäure sind.

4. Verbundfasermembran nach einem der Ansprüche 1 bis 3, bei der die Fasermembran eine gewebte Schicht umfasst.

5. Verbundfasermembran zur Verwendung bei einer Gewebereparatur nach Anspruch 4, bei der die gewebte Schicht aus Polypropylen, Polyvinylidenfluorid, Polytetrafluorethylen, Polyester, Polymilchsäure und Polymilch-co-glykolsäure hergestellt ist.

6. Verfahren zur Herstellung der Fasermembran nach einem der Ansprüche 1 bis 3, umfassend die folgenden Schritte:

(1) Lösen von zwei Fasermaterialien mit verschiedenen Löslichkeitseigenschaften in entsprechenden Lösungsmitteln, so dass zwei Arten von homogenen Fasermateriallösungen erhalten werden;
(2) Einbringen der zwei Arten von homogenen Fasermateriallösungen, die im Schritt (1) erhalten worden sind, in verschiedene elektrostatische Spinninjektionsspritzen zum Erhalten einer Fasermembran, die aus zwei Arten von Faserfilamenten mit unterschiedlichen Löslichkeitseigenschaften, die sich gegenseitig und in einer verschlungenen Weise schneiden, durch elektrostatisches Spinnen ausgebildet wird;
(3) gemäß der Löslichkeitseigenschaften der Materialien der Faserfilamente, Lösen einer Art von Faserfilamenten in der Fasermembran, die im Schritt (2) hergestellt worden ist, mit einem Lösungsmittel, und unverändert Halten der anderen Art von Faserfilamenten, so dass die Fasermembran erhalten wird, die für eine Gewebereparatur verwendet wird.

7. Verfahren zur Herstellung der Fasermembran nach einem der Ansprüche 1 bis 3, umfassend die folgenden Schritte:

(1) Lösen eines Fasermaterials in einem Lösungsmittel, so dass eine homogene Fasermateriallösung erhalten wird;

(2) Einbringen der homogenen Fasermateriallösung, die im Schritt (1) erhalten worden ist, in eine elektrostatische Spinninjektionsspritze, Durchführen eines elektrostatischen Spinnens zum Erhalten von Fasern und Aufnehmen der Fasern zu einer membranartigen Struktur zum Erhalten einer Fasermembran;

(3) Voreinfrieren der im Schritt (2) hergestellten Fasermembran, nachdem sie einem Ultraschallquellen mit einem Lösungsmittel unterzogen worden ist, und dann Durchführen eines Vakuumgefriertrocknens zum Erhalten der Fasermembran, die für eine Gewebereparatur verwendet wird.

8. Verfahren zur Herstellung der Fasermembran nach einem der Ansprüche 1 bis 3, umfassend die folgenden Schritte:

(1) Lösen eines Fasermaterials in einem Lösungsmittel, so dass eine homogene Fasermateriallösung erhalten wird;

(2) Einbringen der homogenen Fasermateriallösung, die im Schritt (1) erhalten worden ist, in eine elektrostatische Spinninjektionsspritze zum Erhalten einer Fasermembran durch elektrostatisches Spinnen;

(3) Strecken der im Schritt (2) hergestellten Fasermembran entlang der Richtung der horizontalen Achse oder der Längsachse der Fasermembran, und nach dem Stoppen des Streckens, Schlichten der Fasermembran in diesem Streckzustand; und dann Strecken der Fasermembran entlang der Richtung senkrecht zu der vorstehend genannten Streckrichtung und nach dem Stoppen des Streckens Schlichten der Fasermembran in diesem Streckzustand zum Erhalten einer flaumigen Faserschicht (A).

9. Verfahren nach einem der Ansprüche 6 bis 8, bei dem während des elektrostatischen Spinnens im Schritt (2) die Rate einer Mikroinjektionspumpe auf 0,1 bis 15,0 ml/Stunde eingestellt wird, die Spannung eines Hochspannungsgenerators auf 5 bis 45 kV eingestellt wird und die Aufnahmedistanz einer Aufnahmevorrichtung auf 5,0 bis 30,0 cm eingestellt ist.

10. Implantierbare Membran zur Verwendung bei einer Gewebereparatur bei der Behandlung einer weiblichen Beckenbodendysfunktion, umfassend eine flaumige Faserschicht (A) mit einer Flaumigkeit von 400 bis 1500 cm$^3$/g und einer Weichheit von 50 bis 500 mN, wobei die flaumige Faserschicht (A) die Fasermembran nach einem der Ansprüche 1 bis 3 ist.

11. Implantierbare Membran zur Verwendung nach Anspruch 10, wobei die implantierbare Membran ferner eine gerichtete Faserschicht (B) umfasst, die eine Schicht mit einer porösen dreidimensionalen Struktur ist, die durch die gerichtete Anordnung von Faserfilamenten gebildet wird und wobei alle Schichten in der implantierbaren Membran mittels elektrostatischen Spinnens oder eines Ultraschallverschmelzens oder einer Naht vereinigt werden.

12. Implantierbare Membran zur Verwendung nach Anspruch 11, bei der die Durchmesser der Faserfilamente in der gerichteten Faserschicht (B) 10 nm bis 20 μm betragen.

13. Implantierbare Membran zur Verwendung nach einem der Ansprüche 10 bis 12, wobei die implantierbare Membran hämostatische Arzneistoffe, antiinfektive Arzneistoffe, einen Zellwachstumsregulator, einen Toxizitätsinhibitor, anästhetische Mikro-Nano-Teilchen und/oder Arzneistoffe zur Behandlung einer Harninkontinenz enthält.

14. Implantierbare Membran zur Verwendung nach einem der Ansprüche 10 bis 13, wobei die Oberfläche der implantierbaren Membran eine hämostatische Schicht enthält, wobei die hämostatische Schicht Kollagen, Polyoxyethylenfasern, Chitosan, Fibrin, Peptid und/oder Thrombin enthält.

15. Implantierbare Membran zur Verwendung nach Anspruch 13 oder Anspruch 14, bei der die hämostatische Schicht durch Verfahren eines elektrostatischen Spinnens, eines Gefriertrocknens, eines Lufttrocknens oder eines Vakuumtrocknens gebildet wird.

16. Implantierbare Membran zur Verwendung nach einem der Ansprüche 10 bis 15, wobei die implantierbare Membran Durchgangslöcher aufweist, die durch die obere und untere Oberfläche der implantierbaren Membran verlaufen.

17. Verfahren zur Herstellung der implantierbaren Membran zur Verwendung nach einem der Ansprüche 10 bis 16, umfassend das Herstellen der flaumigen Faserschicht (A) gemäß dem Verfahren nach einem der Ansprüche 6 bis 9, wobei das Verfahren ferner einen Schritt zur Herstellung der gerichteten Faserschicht (B) durch elektrostatisches Spinnen aufweist.

18. Implantierbare medizinische Vorrichtung zur Verwendung bei der Behandlung einer weiblichen Beckenbodendysfunktion, welche die implantierbare Membran nach einem der Ansprüche 10 bis 16 umfasst.

19. Implantierbare medizinische Vorrichtung zur Verwendung nach Anspruch 18, wobei die implantierbare medizinische Vorrichtung eine spannungsfreie Harninkontinenzschlinge oder ein Beckenbodenfüllstück ist.

20. Implantierbare medizinische Vorrichtung zur Verwendung nach Anspruch 19, wobei die spannungsfreie Harninkontinenzschlinge einen Hauptkörperteil und einen Endteil umfasst, wobei der Hauptkörperteil aus der implantierbaren Membran nach einem der Ansprüche 10 bis 16 hergestellt ist und der Endteil für das Ziehen mit einem chirurgischen Instrument und/oder zum Fixieren verwendet wird.

21. Implantierbare medizinische Vorrichtung zur Verwendung nach Anspruch 20, bei welcher der Hauptkörperteil eine lineare oder gewellte Außenkontur aufweist.

22. Implantierbare medizinische Vorrichtung nach Anspruch 21, wobei das Beckenbodenfüllstück einen zentralen massiven Teil nach einem der Ansprüche 10 bis 16 zur Verwendung bei der Behandlung eines Beckenorganprolaps und -vorwölbens umfasst, und der zentrale massive Teil aus der implantierbaren Membran nach einem der Ansprüche 10 bis 16 hergestellt ist, ferner umfassend eine armartige Struktur, die am Rand des zentralen massiven Teils zum Halten eines prozidenten Beckenorgans angeordnet ist.

23. Nicht-haftende Gewebereparaturmembran, wobei die Oberflächenschicht auf einer Seite der nicht-haftenden Gewebereparaturmembran eine flaumige Faserschicht (A') ist und die Oberflächenschicht auf der anderen Seite eine nicht-haftende Schicht (B') ist; und wobei die flaumige Faserschicht (A') die Fasermembran nach einem der Ansprüche 1 bis 3 ist und die nicht-haftende Schicht (B') Chitosan und/oder Carboxymethylchitosan umfasst.

24. Nicht-haftende Gewebereparaturmembran nach Anspruch 23, die eine Zwischenschicht zwischen der flaumigen Faserschicht (A') und der nicht-haftenden Schicht (B') umfasst, wobei die Zwischenschicht eine gewebte Schicht (C') oder eine Faserschicht (D') oder eine Kombination davon umfasst.

25. Nicht-haftende G ewebereparaturmembran n ach Anspruch 24, bei der die Faserschicht (D') durch Verweben von Faserfilamenten mit Durchmessern von 10 nm bis 100 $\mu$m hergestellt wird und eine poröse Struktur mit einer Flaumigkeit von 200 bis 2000 cm$^3$/g aufweist.

26. Nicht-haftende Gewebereparaturmembran nach Anspruch 24 oder Anspruch 25, bei der die nicht-haftende Schicht (B') mittels Sprühbeschichten oder Ultraschallschweißen mit anderen Schichten kombiniert worden ist.

27. Verfahren zur Herstellung der nicht-haftenden Gewebereparaturmembran nach einem der Ansprüche 23 bis 26, umfassend: Herstellen der flaumigen Faserschicht (A') nach einem der Ansprüche 6 bis 9 und Kombinieren der nicht-haftenden Schicht (B') mit anderen Schichten mittels Sprühbeschichten oder Ultraschallschweißen.

28. Verwendung der Fasermembran nach einem der Ansprüche 1 bis 3 oder der Verbundfasermembran nach Anspruch 4 oder 5 oder der nicht-haftenden Gewebereparaturmembran nach einem der Ansprüche 24 bis 26 bei der Herstellung eines Hernien-Reparaturfüllstücks, eines Behandlungssystems einer weiblichen Beckenbodydysfunktion, einer künstliche Rotatorenmanschette, eines harte Hirnhaut-Reparaturfüllstücks, eines Rückenmarkhaut-Reparaturfüllstücks, von künstlicher Haut, eines Perikard-Füllstücks, von künstlichen Blutgefäßen, von künstlichen Nervenleitungen, eines künstlichen Periodontiums, eines künstlichen Bands, einer künstlichen Achillessehne und von Knochenreparaturprodukten, wobei das Behandlungssystem für eine weibliche Beckenbodendysfunktion ein Beckenboden-Reparaturfüllstück, eine Harninkontinenzschlinge oder ein Fistel-Reparaturfüllstück ist.

29. Hernien-Reparaturfüllstück, Beckenboden-Reparaturfüllstück, Harninkonti-nenzschlinge, Fistel-Reparaturfüllstück, künstliche Rotatorenmanschette, harte Hirnhaut-Reparaturfüllstück, Rückenmarkhaut-Reparaturfüllstück, künstliche Haut, Perikard-Füllstück, künstliche Blutgefäße, künstliche Nervenleitungen, künstliches Periodontium, künstliches Band, künstliche Achillessehne und Knochenreparaturprodukte nach einem der Ansprüche 1 bis 3, 8, 9 oder 24 bis 27.

**Revendications**

1. Membrane fibreuse destinée à être utilisée pour la réparation de tissus, ladite membrane fibreuse étant formée en entrelaçant des filaments fibreux avec des diamètres de 10 nm à 100 $\mu$m, de structure poreuse avec un gonflant de 200 à 2000 cm$^3$/g, dans laquelle la taille moyenne des pores de la membrane fibreuse est de 50 à 500 $\mu$m, l'épaisseur de la membrane fibreuse est de 0,2 à 2 mm, sa résistance à la traction est de 10 à 300 N/cm, et la douceur de la membrane fibreuse est de 50 à 500 mN.

2. Membrane fibreuse selon la revendication 1, dans laquelle les filaments fibreux peuvent être produits à partir de matériaux biodégradables, de matériaux non biodégradables ou leurs combinaisons, lesdits matériaux biodégradables étant des matériaux biodégradables synthétiques ou des matériaux polymères naturels ; et les matériaux non biodégradables étant des matériaux à base de fluoropolymères, des matériaux à base de polypropylène ou des matériaux à base de polyuréthane.

3. Membrane fibreuse selon la revendication 2, dans laquelle les matériaux des filaments fibreux sont un ou plusieurs des matériaux suivants :
   acide polylactique, polycaprolactone, acide polyglycolique, acide polylactique-co-glycolique, polymère 1,3-propanediol, collagène, gélatine, fibrine, fibroine de soie, polymère élastine mimétique peptide, chitosan, chitosan modifié, fluorure de polyvinylidène, polytétrafluoroéthylène, polyuréthane, polycarbonate polyuréthane, polyuréthane à base de polyéther, polyuréthane modifié au silane, polyéthylène glycol, polyéthylène téréphtalate, polyméthyle méthacrylate, poly(3-hydroxybutyrate-co-3-hydroxyvalérate), poly(3-hydroxybutyrate-co-3-hydroxyhexanoate), polyphosphate, anhydride polyamino formique, polyesteramide, alcool polyvinyle, poly(para-dioxanone), polycarbonate, amidon, cellulose, alginate, sulfate de chondroïtine, héparine, glucosan et acide alginique.

4. Membrane fibreuse composite selon l'une quelconque des revendications 1 à 3, dans laquelle la membrane fibreuse comprend une couche tissée.

5. Membrane fibreuse composite destinée à être utilisé pour la réparation de tissus selon la revendication 4, dans laquelle la couche tissée est réalisée à partir de polypropylène, fluorure de polyvinylidène, polytétrafluoroéthylène, polyester, acide polylactique et acide polylactique-co-glycolique.

6. Procédé de préparation de la membrane fibreuse selon l'une quelconque des revendications 1 à 3, comprenant les étapes suivantes consistant à :

   (1) dissoudre deux matériaux fibreux ayant des propriétés de solubilité différentes dans des solvants correspondants pour obtenir deux sortes de solutions homogènes de matériau fibreux ;
   (2) placer les deux sortes de solutions homogènes de matériau fibreux obtenues dans l'étape (1) dans deux seringues d'injection de filature électrostatique différentes pour obtenir une membrane fibreuse formée par deux sortes de filaments fibreux ayant des propriétés de solubilité différentes qui s'entrecroisent mutuellement et de manière imbriquée, par filature électrostatique ;
   (3) selon les propriétés de solubilité des matériaux des filaments fibreux, dissoudre une sorte de filaments fibreux dans la membrane fibreuse produite dans l'étape (2) avec un solvant, et maintenir l'autre sorte de filaments fibreux sans changement pour obtenir la membrane fibreuse utilisée pour la réparation de tissus.

7. Procédé de préparation de la membrane fibreuse selon l'une quelconque des revendications 1 à 3, comprenant les étapes suivantes consistant à :

   (1) dissoudre un matériau fibreux dans un solvant pour obtenir une solution homogène de matériau fibreux ;
   (2) placer la solution homogène de matériau fibreux obtenue dans l'étape (1) dans une seringue d'injection de filature électrostatique, effectuer une filature électrostatique pour obtenir des fibres, et recevoir les fibres dans une structure semblable à une membrane pour obtenir une membrane fibreuse ;
   (3) précongeler la membrane fibreuse préparée dans l'étape (2) après l'avoir soumise à un gonflement par ultrasons avec un solvant, et effectuer ensuite un séchage par congélation sous vide afin d'obtenir la membrane fibreuse utilisée pour la réparation de tissus.

8. Procédé de préparation de la membrane fibreuse selon l'une quelconque des revendications 1 à 3, comprenant les étapes suivantes consistant à :

(1) dissoudre un matériau fibreux dans un solvant pour obtenir une solution homogène de matériau fibreux ;

(2) placer la solution homogène de matériau fibreux obtenu dans l'étape (1) dans une seringue d'injection de filature électrostatique pour obtenir une membrane fibreuse par filature électrostatique ;

(3) étirer la membrane fibreuse préparée dans l'étape (2) le long de la direction de l'axe horizontal ou de l'axe longitudinal de la membrane fibreuse et, après avoir arrêté l'étirage, mettre à la taille la membrane fibreuse dans cet état étiré ; et ensuite étirer la membrane fibreuse le long de la direction perpendiculaire à la direction d'étirage ci-dessus et, après avoir arrêté l'étirage, mettre à la taille la membrane fibreuse dans cet état étiré pour obtenir une couche fibreuse ayant du gonflant (A).

9. Procédé selon l'une quelconque des revendications 6 à 8, dans lequel, pendant l'étape (2) de filature électrostatique, le débit d'une pompe de microinjection est ajusté à 0,1 à 15,0 ml/h, la tension d'un générateur de haute tension est ajustée à 5 à 45 KV, et la distance de réception d'un dispositif de réception est ajustée à 5,0 à 30,0 cm.

10. Membrane implantable destinée à être utilisée pour la réparation de tissus dans le traitement de dysfonctionnements du plancher pelvien chez la femme, comprenant une couche fibreuse ayant du gonflant (A), avec un gonflant de 400 à 1500 cm$^3$/g, et une douceur de 50 à 500 mN, dans laquelle la couche fibreuse présentant du gonflant (A) est la membrane fibreuse selon l'une quelconque des revendications 1 à 3.

11. Membrane implantable selon la revendication 10, dans laquelle la membrane implantable comprend en outre une couche fibreuse directionnelle (B) qui est une couche avec une structure poreuse tridimensionnelle formée par l'agencement directionnel de filaments fibreux et dans laquelle toutes les couches de la membrane implantable sont combinées au moyen d'une filature électrostatique, ou d'une fusion par ultrasons, ou encore d'une suture.

12. Membrane implantable selon la revendication 11, dans laquelle les diamètres des filaments fibreux dans la couche fibreuse directionnelle (B) sont de 10 nm à 20 $\mu$m.

13. Membrane implantable selon l'une quelconque des revendications 10 à 12, dans laquelle la membrane implantable contient des médicaments hémostatiques, des médicaments antiinfection, un régulateur de croissance cellulaire, un inhibiteur de toxicité, des micro/nanoparticules d'anesthésique et/ou des médicaments pour le traitement de l'incontinence urinaire.

14. Membrane implantable selon l'une quelconque des revendications 10 à 13, dans laquelle la surface de la membrane implantable contient une couche hémostatique, ladite couche hémostatique contenant du collagène, des fibres de polyoxyéthylène, du chitosan, de la fibrine, de la peptide et/ou de la thrombine.

15. Membrane implantable selon la revendication 13 ou 14, dans laquelle la couche hémostatique est formée par des procédés incluant la filature électrostatique, le séchage cryogénique, le séchage à l'air ou le séchage sous vide.

16. Membrane implantable selon l'une quelconque des revendications 10 à 15, dans laquelle la membrane implantable comporte des trous traversants qui pénètrent à travers les surfaces supérieure et inférieure de la membrane implantable.

17. Procédé pour préparation de la membrane implantable selon l'une quelconque des revendications 10 à 16, comprenant la préparation de la couche fibreuse présentant du gonflant (A) en accord avec le procédé selon l'une quelconque des revendications 6 à 9, le procédé comprenant en outre une étape consistant à préparer la couche fibreuse directionnelle (B) par filature électrostatique.

18. Dispositif médical implantable destiné à être utilisé dans le traitement de dysfonctionnements du plancher pelvien chez la femme, comprenant la membrane implantable selon l'une quelconque des revendications 10 à 16.

19. Dispositif médical implantable selon la revendication 18, dans lequel le dispositif médical implantable est une écharpe d'incontinence urinaire dépourvue de tension ou un patch pour plancher pelvien.

20. Dispositif médical implantable selon la revendication 19, dans lequel l'écharpe d'incontinence urinaire dépourvue de tension comprend une partie formant corps principal et une partie terminale, la partie formant corps principal est réalisée à partir de la membrane implantable selon l'une quelconque des revendications 10 à 16, et la partie terminale est utilisée pour la traction et/ou la fixation d'un instrument chirurgical.

**21.** Dispositif médical implantable selon la revendication 20, dans lequel la partie formant corps principal a un contour extérieur linéaire ou ondulé.

**22.** Dispositif médical implantable selon la revendication 21, dans lequel le patch de plancher pelvien comprend une partie massive centrale selon l'une quelconque des revendications 10 à 16 destinée à être utilisée dans le traitement du prolapsus et du gonflement des organes pelviens, et la partie massive centrale est réalisée à partir de la membrane implantable selon l'une quelconque des revendications 10 à 16, comprenant en outre une structure semblable à un bras qui est positionnée au niveau de la périphérie de la partie massive centrale pour suspendre une procidence des organes pelviens.

**23.** Membrane anti-adhésion de réparation de tissus, dans laquelle la couche de surface sur un côté de la membrane anti-adhésion de réparation de tissus est une couche fibreuse présentant du gonflant (A'), et la couche de surface sur l'autre côté est une couche anti-adhésion (B') ; et dans laquelle la couche fibreuse présentant du gonflant (A') est la membrane fibreuse selon l'une quelconque des revendications 1 à 3, et la couche anti-adhésion (B') contient du chitosan et/ou du chitosan carboxy-méthyle.

**24.** Membrane anti-adhésion de réparation de tissus selon la revendication 23, comprenant une couche intermédiaire entre la couche fibreuse présentant du gonflant (A') et la couche anti-adhésion (B'), dans laquelle ladite couche intermédiaire comprend une couche tissée (C'), ou une couche fibreuse (D'), ou une combinaison de celles-ci.

**25.** Membrane anti-adhésion de réparation de tissus selon la revendication 24, dans laquelle la couche fibreuse (D') est formée en entrelaçant des filaments fibreux avec des diamètres de 10 nm à 100 $\mu$m, et présente une structure poreuse, avec un gonflant de 200 à 2000 cm$^3$/g.

**26.** Membrane anti-adhésion de réparation de tissus selon la revendication 24 ou 25, dans laquelle la couche anti-adhésion (B') est combinée avec d'autres couches au moyen d'un revêtement par pulvérisation ou d'un soudage électronique.

**27.** Procédé de préparation de la membrane anti-adhésion de réparation de tissus selon l'une quelconque des revendications 23 à 26, comprenant les étapes consistant à : préparer la couche fibreuse présentant du gonflant (A') selon l'une quelconque des revendications 6 à 9, et combiner la couche anti-adhésion (B') avec d'autres couches au moyen d'un revêtement par pulvérisation ou d'un soudage électronique.

**28.** Utilisation de la membrane fibreuse selon l'une quelconque des revendications 1 à 3, ou de la membrane fibreuse composite selon les revendications 4 ou 5, ou de la membrane anti-adhésion de réparation de tissus selon l'une quelconque des revendications 24 à 26, dans la préparation d'un patch de réparation pour hernie, d'un système de traitement des dysfonctionnements du plancher pelvien chez la femme, d'une coiffe de rotateur artificielle, d'un patch de réparation de duremère, d'un patch de réparation des méninges spinales, de peau artificielle, d'un patch du péricarde, de vaisseaux sanguins artificiels, de conduits nerveux artificiels, de périodonte artificiel, de ligaments artificiels, de tendons d'Achille artificiels, et de produits de réparation osseuse, dans laquelle le système de traitement pour dysfonctionnements du plancher pelvien chez la femme est un patch de réparation de plancher pelvien, une écharpe pour incontinence urinaire, ou un patch de réparation de fistules.

**29.** Patch de réparation pour hernie, patch de réparation de plancher pelvien, écharpe pour incontinence urinaire, patch de réparation de fistules, coiffe de rotateur artificielle, patch de réparation de duremère, patch de réparation de méninges spinales, peau artificielle, patch pour péricarde, vaisseaux sanguins artificiels, conduits nerveux artificiels, parodonte artificiel, ligaments artificiels, tendon d'Achille artificiel, et produits de réparation osseuse selon l'une quelconque des revendications 1 à 3, 8, 9 ou 24 à 27.

Fig. 1

Fig. 2

Fig. 3

Fig. 4

Fig. 5

Fig. 6

Fig. 7

Fig. 8

Fig. 9

Leftside
PCU
material

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

Fig. 23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011003422 A1